(19) Europäisches Patentamt
European Patent Office
Office européen des brevets

(11) EP 3 189 851 A1

(12) **EUROPEAN PATENT APPLICATION**
published in accordance with Art. 153(4) EPC

(43) Date of publication:
**12.07.2017 Bulletin 2017/28**

(21) Application number: **15828961.1**

(22) Date of filing: **04.08.2015**

(51) Int Cl.:
***A61K 39/00*** *(2006.01)* ***A61K 39/39*** *(2006.01)*
***A61K 45/00*** *(2006.01)* ***A61P 31/00*** *(2006.01)*
***A61P 31/12*** *(2006.01)* ***A61P 35/00*** *(2006.01)*
***A61P 43/00*** *(2006.01)*

(86) International application number:
**PCT/JP2015/072109**

(87) International publication number:
**WO 2016/021605 (11.02.2016 Gazette 2016/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **04.08.2014 JP 2014159002**

(71) Applicant: **Nitto Denko Corporation**
**Ibaraki-shi, Osaka 567-8680 (JP)**

(72) Inventors:
- **MAEDA, Yoshiki**
  **Ibaraki-shi**
  **Osaka 567-8680 (JP)**
- **OKAZAKI, Arimichi**
  **Ibaraki-shi**
  **Osaka 567-8680 (JP)**
- **ASARI, Daisuke**
  **Ibaraki-shi**
  **Osaka 567-8680 (JP)**
- **HORI, Mitsuhiko**
  **Ibaraki-shi**
  **Osaka 567-8680 (JP)**

(74) Representative: **Müller-Boré & Partner Patentanwälte PartG mbB**
**Friedenheimer Brücke 21**
**80639 München (DE)**

# (54) VACCINE PHARMACEUTICAL COMPOSITION FOR SUPPRESSING APOPTOSIS OF CTL OR INHIBITING SUPPRESSION OF INDUCTION OF CTL

(57) The present invention aims to provide a vaccine pharmaceutical composition that can be universally used for induction of cellular immunity to various antigens and that exerts a high inducing effect. The present invention relates to a vaccine pharmaceutical composition that suppresses apoptosis of CTL or inhibits suppression of induction of CTL.

EP 3 189 851 A1

## Description

TECHNICAL FIELD

The present invention relates to suppression of induction of apoptosis of antigen-specific CD8-positive T cells.

BACKGROUND ART

Vaccines that are generally widely used are made from pathogens such as microorganisms or viruses or prepared by partially weakening or destroying toxins of pathogens. Vaccines are administered to living bodies to induce immunity to prevent infectious diseases. Also, cancer vaccines have been developed for the purpose of inducing specific attack of the cellular immune system to cancer cells by allowing a cellular immune mechanism to recognize a cancer cell-specific antigen. These cancer vaccines are used as one of the means of treating cancer.

In the cancer treatment, it is known to use an adjuvant in order to enhance an effect of a cancer vaccine that induces cellular immunity. Examples of adjuvants include 1H-imidazo[4,5-c]quinoline-4-amine, imiquimod (e.g., see Patent Literature 1), cyclic dinucleotide analogues (e.g., see Patent Literature 2 and Patent Literature 3) such as cyclic diGMP (c-di-GMP), and TLR2, 3, 7, 8, and 9 ligands (e.g., see Patent Literature 4).

In cellular immunity-based treatment of diseases such as cancer, it is necessary to continuously induce antigen-specific CD8-positive T cells. Thus, the treatment usually requires repeated immunotherapies . However, it has been reported that repeated injections of an antigen or adjuvant results in high expression of PD-1 receptor, which is an immunosuppressive receptor, in antigen-specific CD8-positive T cells (for example, see "Non Patent Literature 1"). The PD-1 receptor is known to be recognized by antigen presenting cells and cancer cells PD-L1, to induce programmed cell death (apoptosis) of CD8-positive T cells, and to reduce induction of antigen-specific CD8-positive T cells. In other words, as described above, effective induction of antigen-specific CD8-positive T cells cannot be achieved simply by administering an antigen and an adjuvant.

In addition to the above, it has also been reported that repeated injections of an antigen or adjuvant reduce antigen-specific CD8-positive T cells (for example, see Non Patent Literature 2). As described above, effective induction of antigen-specific CD8-positive T cells cannot be achieved simply by administering an antigen and an adjuvant.

CITATION LIST

- Patent Literature

Patent Literature 1: JP-T H07-505883
Patent Literature 2: JP-T 2007-529531
Patent Literature 3: US 2008/0286296
Patent Literature 4: US 2008/0193487

Non Patent Literature

Non Patent Literature 1: Daisuke Muraoka et al., The Journal of Immunology, 185, 3768-3776 (2010)
Non Patent Literature 2: Yoshihiro Oka et al., Current Opinion in Immunology, 20: 211-220 (2008)

SUMMARY OF INVENTION

- Technical Problem

In view of the above-described situation, the present invention aims to provide a vaccine pharmaceutical composition that is universally effective in induction of cellular immunity to various antigens and that can effectively induce antigen-specific CD8-positive T cells in continuous administration.

- Solution to Problem

The present inventors focused on suppression of expression of PD-1 receptor in antigen-specific CD8-positive

T cells as a means to effectively and continuously induce antigen-specific CD8-positive T cells in multiple dose administration. As a result of extensive studies, the present inventors identified the type of cellular immunity induction promoter and found that it is possible to suppress expression of PD-1 receptor in antigen-specific CD8-positive T cells by controlling the dose ratio between an antigen and a cellular immunity induction promoter to be administered. In other words, the present inventors found that it is possible to suppress induction of apoptosis of CD8-positive T cells.

The present inventors also focused on helper T cells among various cells that are involved in cellular immunity, especially on the balance between Th1 cell count and Treg cell count, as a means to effectively and continuously induce antigen-specific CD8-positive T cells in multiple dose administration. Th1 cells enhance cellular immunity, and Treg cells suppress cellular immunity. As a result of extensive studies with the focus on the balance in the number of these cells, the present inventors found that an increase in the ratio of Th1 cell count to Treg cell count enhances induction of cellular immunity even in multiple dose administration. Specifically, the present inventors identified the type of cellular immunity induction promoter and found that it is possible to control the balance between Th1 cell count and Treg cell count by controlling the dose ratio between an antigen and a cellular immunity induction promoter.

Specifically, the present invention relates to a vaccine pharmaceutical composition for administration containing an antigen to induce cellular immunity, wherein the vaccine pharmaceutical composition suppresses apoptosis of CTL or inhibits suppression of induction of CTL.

In addition, with the vaccine pharmaceutical composition of the present invention, the expression rate of PD-1 receptor in antigen-specific CD8-positive T cells in an animal model for immunological evaluation administered with the vaccine pharmaceutical composition is preferably 40% or less. In addition, with the vaccine pharmaceutical composition of the present invention, the rate of induction of apoptosis of antigen-specific CD8-positive T cells in an animal model for immunological evaluation administered with the vaccine pharmaceutical composition of the present invention is preferably 30% or less.

In addition, with the vaccine pharmaceutical composition of the present invention, the antigen-specific Th1/Treg ratio in an animal model for immunological evaluation administered with the vaccine pharmaceutical composition is preferably controlled.

In addition, with the vaccine pharmaceutical composition of the present invention, the antigen-specific Th1/Treg ratio in an animal model for immunological evaluation administered with the vaccine pharmaceutical composition is preferably controlled to 0.001% or more, 0.01% or more, 0.1% or more, 1% or more, 10% or more, or 30% or more.

In addition, the vaccine pharmaceutical composition of the present invention preferably further contains a first cellular immunity induction promoter.

In addition, the vaccine pharmaceutical composition of the present invention is preferably administered in multiple doses.

In addition, the first cellular immunity induction promoter in the vaccine pharmaceutical composition of the present invention is preferably at least one of a TLR ligand or a cyclooxygenase inhibitor.

In addition, the vaccine pharmaceutical composition of the present invention preferably further contains a second cellular immunity induction promoter which is a helper peptide.

The present invention will be described in detail below.

The terms used in the present specification will be defined so that the vaccine pharmaceutical composition of the present invention can be more easily understood. The terms not defined herein have the meaning which is normally understood by skilled people, particularly those in the fields of medicine, pharmacy, immunology, cell biology, biochemistry, polymer chemistry, and the like, unless the context requires otherwise.

The vaccine pharmaceutical composition of the present invention is a vaccine pharmaceutical composition for administration containing an antigen to induce cellular immunity, wherein the vaccine pharmaceutical composition suppresses apoptosis of CTL or inhibits suppression of induction of CTL. The term "CTL" refers to a cytotoxic T-cell or cytotoxic T-cells.

The vaccine pharmaceutical composition of the present invention contains at least one antigen.

The term "antigen" refers to any substance that can induce immune response. The antigen is not particularly limited, and examples thereof include proteins and peptides. In the case of transdermal administration in which an antigen is required to be skin permeable, use of an antigen having a small molecular weight is preferred. For example, a peptide having about 8 to about 12 amino acid residues can be used.

The antigen is not particularly limited, and examples thereof include a cancer antigen peptide and a peptide derived from an infectious pathogen.

As used herein, the term "cancer" refers to the abnormal expression of oncogene. Examples of the cancer include a cancer associated with overexpression of a gene, such as a hematopoietic tumor or solid cancer.

As used herein, the term "abnormal expression of a gene" means that the expression level of a gene in a cell is significantly increased or decreased by, for example, at least two times or at least four times, as compared to another cell in the same tissue.

As used herein, the term "overexpression" means an increase in the expression level of abnormal expression.

The expression level of a gene can be easily measured by any method known in the relevant technical field.

Examples of the oncogene include survivin gene, GPC3 gene, HER2/neu gene, MAGE-3 gene, MAGE-A1 gene, MAGE-A3/A6 gene, MAGE-A4 gene, MAGE-12 gene, proteinase-3 gene, AFP gene, CA-125 gene, CD44 gene, CEA gene, c-Kit gene, c-met gene, c-myc gene, L-myc gene, COX2 gene, CyclinD1 gene, Cytokeratin-7 gene, Cytokeratin-19 gene, Cytokeratin-20 gene, E2F1 gene, E2F3 gene, EGFR gene, Gli1 gene, hCGβ gene, HIF-1α gene, HnRNP A2/B1 gene, hTERT gene, MDM gene, MDR-1 gene, MMP-2 gene, MMP-9 gene, Muc-1 gene, Muc-4 gene, Muc-7 gene, NSE gene, ProGRP gene, PSA gene, RCAS1 gene, SCC gene, thymoglobulin gene, VEGF-A gene, and VEGF-A gene.

Non-limiting examples of cancers associated with abnormal expression of the survivin gene include malignant lymphoma, bladder cancer, lung cancer, and bowel cancer. Non-liming examples of cancers associated with abnormal expression of the GPC3 gene include liver cancer, bile duct cancer, and stomach cancer. Non-liming examples of cancers associated with abnormal expression of the HER2/neu gene include breast cancer, stomach cancer, ovarian cancer, uterine cancer, bladder cancer, non-small cell lung cancer, and prostatic cancer. Non-liming examples of cancers associated with abnormal expression of the MAGE-3 gene include melanoma, lung cancer, head and neck cancer, bladder cancer, stomach cancer, esophageal cancer, and liver cancer. Non-liming examples of cancers associated with abnormal expression of the proteinase-3 gene include acute myelocytic leukemia and pancreatic cancer.

As used herein, the term "cancer antigen" refers to a substance such as a protein or peptide which is specifically expressed in tumor cells or cancer cells and capable of inducing cellular immune response. Specifically, it refers to a protein expressed based on the oncogene and a partial peptide derived therefrom.

As used herein, the term "cancer antigen peptide" refers to a partial peptide derived from a cancer antigen protein, capable of inducing cellular immune response. Usually, a cancer antigen peptide is produced by decomposition of a cancer antigen protein (which is an oncogene product) in a cancer cell, and is presented on the surface of a cancer cell by MHC class I molecules.

The cancer antigen peptide may be an endogenous cancer antigen peptide isolated and purified from cancer cells, or may be a synthetic peptide having the same amino acid sequence as the endogenous cancer antigen peptide. Specifically, preferred examples of the cancer antigen peptide include survivin 2B peptide, GPC3 peptide, HER2/neu_A24 peptide, MAGE3_A24 peptide, PR1 peptide, HER2/neu_A02 peptide, MAGE3_A02 peptide, HER2/neu_E75 peptide, MUC1 peptide, and altered peptides thereof.

As used herein, the term "survivin 2B peptide" refers to a peptide derived from survivin which is an oncogene product, having a sequence of Ala Tyr Ala Cys Asn Thr Ser Thr Leu (SEQ ID No: 1).

As used herein, the term "GPC3 peptide" refers to a peptide derived from GPC3 which is an oncogene product, having a sequence of Glu Tyr Ile Leu Ser Leu Glu Glu Leu (SEQ ID No: 2).

As used herein, the term "HER2/neu_A24 peptide" refers to an HLA-A24-restricted peptide derived from HER2/neu which is an oncogene product, having a sequence of Thr Tyr Leu Pro Thr Asn Ala Ser Leu (SEQ ID No: 3).

As used herein, the term "MAGE3_A24 peptide" refers to an HLA-A24-restricted peptide derived from MAGE-3 which is an oncogene product, having a sequence of Ile Met Pro Lys Ala Gly Leu Leu Ile (SEQ ID No: 4).

As used herein, the term "PR1 peptide" refers to a peptide derived from proteinase-3 which is an oncogene product, having a sequence of Val Leu Gln Glu Leu Asn Val Thr Val (SEQ ID No: 5).

As used herein, the term "HER2/neu_A02 peptide" refers to an HLA-A02-restricted peptide derived from HER2/neu which is an oncogene product, having a sequence of Lys Val Phe Gly Ser Leu Ala Phe Val (SEQ ID No: 6).

As used herein, the term "MAGE3_A02 peptide" refers to an HLA-A02-restricted peptide derived from MAGE-3 which is an oncogene product, having a sequence of Lys Val Ala Glu Ile Val His Phe Leu (SEQ ID No: 7).

As used herein, the term "HER2/neu_E75 peptide" refers to a peptide derived from a product (HER2 protein) of an oncogene HER2/neu, having a sequence of Lys Ile Phe Gly Ser Leu Ala Phe Leu (SEQ ID No: 8).

As used herein, the term "MUC1 peptide" refers to a peptide derived from MUC1 protein which is a glycoprotein that is highly expressed on many cancer cells, having a sequence of Ser Thr Ala Pro Pro Val His Asn Val (SEQ ID No: 9).

As used herein, the term "altered peptide" refers to a peptide in which all or a part of amino acids are altered by, for example, substitution or modification.

The altered peptide is not particularly limited, and examples thereof include peptides such as: (a) a peptide having an amino acid sequence in which one to several amino acids (for example, 1, 2, 3, 4, or 5 amino acids) are substituted, deleted, or added in the amino acid sequence of the peptide; and (b) a peptide having an amino acid sequence in which all or a part of amino acids (for example, 1, 2, 3, 4, 5, 6, 7, 8, 9, or 10 amino acids) are modified in the amino acid sequence of the peptide.

The amino acids of the altered peptide may be modified in any manner. Examples of such modifications include acetylation; alkylation such as methylation; glycosylation; hydroxylation; carboxylation; aldehydation; phosphorylation; sulfonylation; formylation; aliphatic chain addition modification such as myristoylation, palmitoylation, and stearoylation; octanoylation; esterification; amidation; deamidation; disulfide bond formation modification such as cystine modification, glutathione modification, and thioglycolic acid modification; glycation; ubiquitination; succinimide formation; glutamylation; and prenylation.

The altered peptide may contain substitution, deletion, or addition of one or more amino acids and modification of one or more amino acids in combination.

As used herein, the term "infectious pathogen-derived antigen" refers to an infectious pathogen or its component or a substance derived from an infectious pathogen or its component, capable of inducing cellular immune response. Thus, it is possible to treat or prevent an infectious disease by administering the infectious pathogen-derived antigen together with the first cellular immunity induction promoter to a subject.

In a preferred embodiment of the present invention, for example, IPEP87 peptide, HBVenv peptide, or an altered peptide of IPEP87 peptide or HBVenv peptide can be used as the infectious pathogen-derived antigen.

As used herein, the term "IPEP87 peptide" refers to a peptide derived from a hepatitis C virus (HCV) protein, having a sequence of Asp Leu Met Gly Tyr Ile Pro Ala Val (SEQ ID No: 10).

As used herein, the term "HBVenv peptide" refers to a peptide derived from a hepatitis B virus (HBV) protein, having a sequence of Trp Leu Ser Leu Leu Val Pro Phe Val (SEQ ID No: 11).

As used herein, the term "infectious disease" refers to a disease caused by infection with an infectious pathogen, growth of an infectious pathogen, or the like.

The infectious disease is not particularly limited, and examples thereof include virus diseases caused by infection with viruses such as adenovirus (e.g., human adenovirus), herpesvirus (e.g., herpes simplex virus, varicella-zoster virus, cytomegalovirus, human herpesvirus, or Kaposi sarcoma-associated herpesvirus), picornavirus (e.g., polio virus, common cold virus, or hepatitis A virus), pox virus (e.g., smallpox virus, vaccinia virus, or molluscum contagiosum virus), picornavirus (e.g., rhinovirus or enterovirus), orthomyxoviridae (e.g., influenza virus), paramyxovirus (e.g., parainfluenza virus, mumps virus, measles virus, respiratory syncytial virus (RSV), or Newcastle disease virus), parvovirus (e.g., adeno associated virus), togavirus (e.g., rubella virus), coronavirus (e.g., SARS coronavirus), hepadnavirus (e.g., hepatitis B virus), flavivirus (e.g., Japanese encephalitis virus, yellow fever virus, dengue virus, West Nile fever, St. Louis encephalitis virus, Murray Valley encephalitis virus, hepatitis C virus, or hepatitis G virus), hepevirus (e.g., hepatitis E virus), papillomavirus (e.g., human papilloma virus), calicivirus (e.g., Norovirus), rhabdovirus (e.g., rabies virus or vesicular stomatitis virus), filovirus (e.g., Ebola hemorrhagic fever virus), arenavirus (e.g., Lassa virus or hepatitis D virus), bunyavirus (e.g., California encephalitis virus or Rift Valley fever virus), reovirus (e.g., rotavirus), or retrovirus (e.g., human immunodeficiency virus (HIV) or adult T-cell leukemia virus); bacterial diseases such as those caused by infection with a bacterium such as Escherichia, Enterobacter, Salmonella, Staphylococcus, dysentery bacillus, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campyrobacter, Vibrio, Serratia, Providencia, Chromobacterium, Brucella, Yersinia, Haemophilus, or Bordetella; fungous diseases such as Chlamydia, candidiasis, aspergillosis, histoplasmosis, and cryptococcal meningitis; malaria; Pneumocystis carinii pneumonia; leishmaniasis; cryptosporidiosis; toxoplasmosis; and Trypanosoma infection.

The peptides mentioned above can be in the free form or any pharmacologically acceptable salt form.

Examples include acid salts (e.g., acetate, TFA salt, hydrochloride, sulfate, phosphate, lactate, tartrate, maleate, fumarate, oxalate, hydrobromate, succinate, nitrate, malate, citrate, oleate, palmitate, propionate, formate, benzoate, picrate, benzenesulfonate, dodecylsulfate, methanesulfonate, p- toluenesulfonate, glutarate, and various amino acid salts), metal salts (e.g., alkali metal salts (e.g., sodium salt and potassium salt), alkaline-earth metal salts (e.g., calcium salt and magnesium salt), and aluminum salt), and amine salts (e.g., triethylamine salt, benzylamine salt, diethanolamine salt, t-butylamine salt, dicyclohexylamine salt, arginine salt, dimethylammonium salt, and ammonium salt). In particular, acetate or TFA salt is preferred.

The peptides mentioned above can be synthesized or produced, isolated, and purified by a known method.

The vaccine pharmaceutical composition of the present invention is a vaccine composition for administration containing any of the above antigens to induce cellular immunity, wherein the expression rate of PD-1 receptor in antigen-specific CD8-positive T cells in an animal model for immunological evaluation administered with the composition is preferably 40% or less, more preferably in the range of 20% to 40%.

In addition, with the vaccine pharmaceutical composition of the present invention, the rate of induction of apoptosis of antigen-specific CD8-positive T cells in an animal model for immunological evaluation administered with the vaccine pharmaceutical composition is preferably 30% or less. Thereby, the vaccine pharmaceutical composition can effectively induce cellular immunity.

The rate of induction of apoptosis of antigen-specific CD8-positive T cells can be measured by an immunity induction experiment using an animal model for immunological evaluation and also by flow cytometry (FACS) with a tetramer that recognizes antigen-specific CD8-positive T cells. A spleen or a lymph node in an animal model for immunological evaluation is used as a sample for measurement of the rate of induction of apoptosis of antigen-specific CD8-positive T cells.

The expression rate of PD-1 receptor in antigen-specific CD8-positive T cells can also be measured by FACS mentioned above. A spleen or a lymph node in an animal model for immunological evaluation is used as a sample for

measurement of the expression rate of PD-1 receptor in antigen-specific CD8-positive T cells.

The vaccine pharmaceutical composition of the present invention controls an antigen-specific Th1/Treg ratio in an animal model for immunological evaluation administered with the vaccine pharmaceutical composition. For example, the Th1/Treg ratio is 0.001% or more, 0.01% or more, 0.1% or more, 1% or more, 10% or more, or 30% or more. Th1 cells enhance cellular immunity. A higher Th1/Treg ratio is preferred. The most preferred Th1/Treg ratio is 100%.

The antigen-specific Th1/Treg ratio can be measured by FACS mentioned above. A spleen or a lymph node in an animal model for immunological evaluation is used as a sample for measurement of the antigen-specific Th1/Treg ratio.

Preferably, the vaccine pharmaceutical composition of the present invention further contains a first cellular immunity induction promoter.

As used herein, the term "cellular immunity induction promoter" refers to any substance that can improve the efficiency to induce cellular immunity to an antigen administered with the substance, as compared to the same efficiency without the substance. Although the substance is not limited by the mechanism of action that promotes induction of cellular immunity, the term refers to those specified herein.

Preferred examples of the first cellular immunity induction promoter include TLR ligands such as TLR1/2 ligand, TLR2 and Dectin-1 ligand, TLR2/6 ligand, TLR3 ligand, TLR5 ligand, TLR7 and/or TLR8 ligand, and TLR9 ligand; cyclic dinucleotides such as cyclic diGMP and cyclic diAMP; immunomodulatory small molecule drugs such as bestatin, pidotimod, and levamisole hydrochloride; cyclooxygenase inhibitors such as etodolac and loxoprofen; prostaglandin receptor antagonists such as EP2 receptor antagonist, EP4 receptor antagonist, DP receptor antagonist, and IP receptor antagonist; prostaglandin receptor agonists such as EP3 receptor agonist; TSLP production inhibitors such as berberine chloride and naringenin; adenylate cyclase inhibitors such as 2',5'-dideoxyadenosine and niacin; omega-3 fatty acids such as eicosapentaenoic acid and docosahexaenoic acid; PPAR agonists such as PPAR-$\alpha$ agonist, PPAR-$\delta$ agonist, and PPAR-$\gamma$ agonist; dopamine receptor antagonists such as D1 receptor antagonist and D5 receptor antagonist; dopamine receptor agonists such as D2 receptor agonist, D3 receptor agonist, and D4 receptor agonist; histamine receptor antagonists such as H1 receptor antagonist and H2 receptor antagonist; histamine receptor agonists such as H1 receptor agonist, H3 receptor agonist, and H4 receptor agonist; serotonin receptor antagonists such as 5-HT2 receptor antagonist, 5-HT4 receptor antagonist, 5-HT6 receptor antagonist, and 5-HT7 receptor antagonist; serotonin receptor agonists such as 5-HT1 receptor agonist and 5-HT2 receptor agonist; vasopressin receptor antagonists such as V2 receptor antagonist; vasopressin receptor agonists such as V1 receptor agonist; muscarinic receptor antagonists such as M1 receptor antagonist, M3 receptor antagonist, and M5 receptor antagonist; muscarinic receptor agonists such as M1 receptor agonist, M2 receptor agonist, M3 receptor agonist, M4 receptor agonist, and M5 receptor agonist; adrenergic receptor antagonists such as $\alpha$1 receptor antagonist, $\beta$1 receptor antagonist, $\beta$2 receptor antagonist, and $\beta$3 receptor antagonist; adrenergic receptor agonists such as $\alpha$1 receptor agonist and $\alpha$2 receptor agonist; angiotensin receptor agonists such as AT2 receptor agonist; GABA receptor agonists such as $GABA_B$ receptor agonist; thrombin receptor antagonists such as PAR-1 receptor antagonist; thrombin receptor agonists such as PAR-1 receptor agonist; opioid receptor agonists such as buprenorphine; leukotriene receptor antagonists such as CysLT1 receptor antagonist and CysLT2 receptor antagonist; leukotriene receptor agonists such as BLT receptor agonist; ADP receptor agonists such as adenosine diphosphate; melatonin receptor agonists such as melatonin; somatostatin receptor agonists such as octreotide; cannabinoid receptor agonists such as dronabinol; sphingosine-1 phosphate receptor agonists such as fingolimod; metabotropic glutamate receptor agonists such as mGluR2 receptor agonist, mGluR3 receptor agonist, mGluR4 receptor agonist, mGluR6 receptor agonist, mGluR7 receptor agonist, and mGluR8 receptor agonist; phospholipase A2 inhibitors such as glycyrrhizic acid; TGF-$\beta$ production inhibitors such as pirfenidone; and Th2 cytokine inhibitors such as suplatast tosylate. In particular, the first cellular immunity induction promoter is preferably a TLR ligand and/or a cyclooxygenase inhibitor. The first cellular immunity induction promoter in the transdermal administration is more preferably a TLR7 ligand.

As used herein, the term "cyclooxygenase inhibitor" refers to a substance that inhibits the function of cyclooxygenase (COX). Hereinafter, the "cyclooxygenase inhibitor" is also referred to as "COX inhibitor". Some COX inhibitors selectively act on a specific cyclooxygenase (e.g., COX-1 or COX-2), and some have no such selectivity. Examples of the COX inhibitor that can be used in the present invention include etodolac, loxoprofen, celecoxib, valdecoxib, parecoxib, lumiracoxib, meloxicam, tenoxicam, diclofenac, mefenamic acid, tolfenamic acid, flufenamic acid, meclofenamic acid, niflumic acid, benzydamine, indobufen, triflusal, tolmetin, fenoprofen, tiaprofenic acid, felbinac, nepafenac, amfenac, pravadoline, zaltoprofen, sulindac, nabumetone, diflunisal, piroxicam, ibuprofen, naproxen, fenoprofen, aspirin, methyl salicylate, salicylamide, salsalate, aloxiprin, tolmetin, indomethacin, proglumetacine, acemetacin, flurbiprofen, pranoprofen, acetaminophen, floctafenine, lornoxicam, tenoxicam, tiaprofenic acid, oxaprozin, ketoprofen, dexketoprofen, dexibuprofen, alminoprofen, ketorolac, mofezolac, phenylbutazone, oxyphenylbutazone, ketophenylbutazone, feprazone, sulfinbutazone, ethenzamide, tiaramide, tinoridine, epirizole, emorfazone, and derivatives thereof, as well as pharmacologically acceptable salts thereof. In particular, etodolac and/or loxoprofen represented by the following formula is preferred.

The loxoprofen is represented by the following formula.

[Chemical Formula 1]

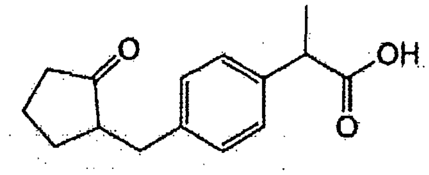

As used herein, the term "prostaglandin receptor antagonist" refers to a substance having a function to prevent prostaglandin from acting on receptors.
The prostaglandin receptor antagonist is not particularly limited, and examples thereof include an EP2 receptor antagonist, an EP4 receptor antagonist, a DP receptor antagonist, and an IP receptor antagonist.
As used herein, the term "EP2 receptor antagonist" refers to a substance having a function to prevent prostaglandin E2 from acting on EP2 receptors.
The EP2 receptor antagonist is not particularly limited, and examples thereof include AH6809 and derivatives thereof, as well as pharmacologically acceptable salts thereof.
The AH6809 is represented by the following formula.

[Chemical Formula 2]

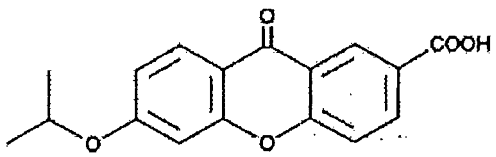

As used herein, the term "EP4 receptor antagonist" refers to a substance having a function to prevent prostaglandin $E_2$ from acting on EP4 receptors.
The EP4 receptor antagonist is not particularly limited, and examples thereof include GW627368X and derivatives thereof, as well as pharmacologically acceptable salts thereof.
The GW627368X is represented by the following formula.

[Chemical Formula 3]

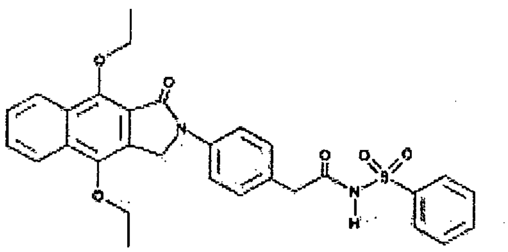

As used herein, the term "DP receptor antagonist" refers to a substance having a function to prevent prostaglandin $D_2$ from acting on DP receptors.
The DP receptor antagonist is not particularly limited, and examples thereof include S-5751, BWA868C, and derivatives thereof, as well as pharmacologically acceptable salts thereof.
The BWA868C is represented by the following formula.

[Chemical Formula 4]

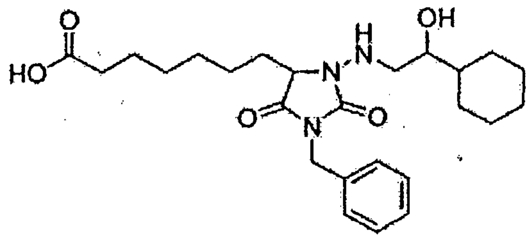

As used herein, the term "IP receptor antagonist" refers to a substance having a function to prevent prostaglandin $I_2$ from acting on IP receptors.

The IP receptor antagonist is not particularly limited, and examples thereof include RO1138452 and derivatives thereof, as well as pharmacologically acceptable salts thereof.

The RO1138452 is represented by the following formula.

[Chemical Formula 5]

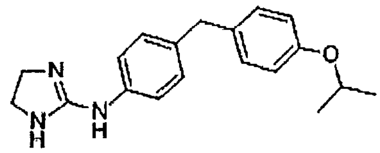

As used herein, the term "prostaglandin receptor agonist" refers to a substance having a function to act on prostaglandin receptors.

The prostaglandin receptor agonist is not particularly limited, and examples thereof include an EP3 receptor agonist.

As used herein, the term "EP3 receptor agonist" refers to a substance having a function to act on EP3 receptors.

The EP3 receptor agonist is not particularly limited, and examples thereof include sulprostone, GR63799, cloprostenol, ONO-AE-248, carbacyclin, and derivatives thereof, as well as pharmacologically acceptable salts thereof.

The sulprostone is represented by the following formula.

[Chemical Formula 6]

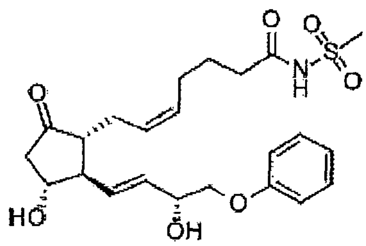

As used herein, the term "TSLP production inhibitor" refers to a substance having a function to suppress the production of TSLP.

The TSLP production inhibitor is not particularly limited, and examples thereof include naringenin, berberine, resveratrol, luteolin, apigenin, chrysoeriol, velutin, rutin, hesperidin, quercetin, daidzein, genistein, and derivatives thereof, as well as pharmacologically acceptable salts thereof.

The berberine is represented by the following formula.

[Chemical Formula 7]

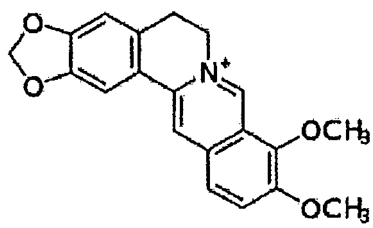

As used herein, the term "adenylate cyclase inhibitor" refers to a substance having a function to suppress the activity of adenylate cyclase.

The adenylate cyclase inhibitor is not particularly limited, and examples thereof include 2',5'-dideoxyadenosine, niacin, insulin, and derivatives thereof, as well as pharmacologically acceptable salts thereof.

The 2',5'-dideoxyadenosine is represented by the following formula.

[Chemical Formula 8]

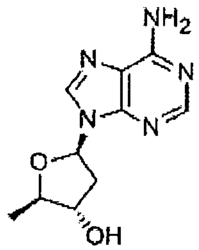

As used herein, the term "omega-3 fatty acid" is one category of unsaturated fatty acids, and refers to a fatty acid having a carbon-carbon double bond in the ω-3 position.

The omega-3 fatty acid is not particularly limited, and examples thereof include eicosapentaenoic acid, α-linolenic acid, docosahexaenoic acid, and derivatives thereof, as well as pharmacologically acceptable salts thereof.

The eicosapentaenoic acid is represented by the following formula.

[Chemical Formula 9]

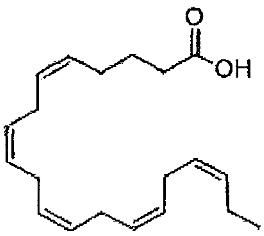

As used herein, the term "PPAR agonist" refers to a substance having a function to act on peroxisome proliferator-activated receptors.

The PPAR agonist is not particularly limited, and examples thereof include PPAR-α agonist, PPAR-δ agonist, and PPAR-γ agonist.

As used herein, the term "PPAR-α agonist" refers to a substance having a function to act on α-type peroxisome proliferator-activated receptors. The term "PPAR-δ agonist" refers to a substance having a function to act on 5-type peroxisome proliferator-activated receptors. The term "PPAR-γ agonist" refers to a substance having a function to act on α-type peroxisome proliferator-activated receptors.

PPAR-α agonist and/or PPAR-δ agonist and/or PPAR-γ agonist is not particularly limited, and examples thereof include clofibrate, fenofibrate, bezafibrate, ciprofibrate, etofibrate, leukotriene B4, telmisartan, oleyl ethanolamide, tetradecylthioacetic acid, troglitazone, pioglitazone, rosiglitazone, balaglitazone, rivoglitazone, ciglitazone, darglitazone, edaglitazone, netoglitazone, indeglitazar, tesaglitazar, muraglitazar, aleglitazar, and derivatives thereof, as well as pharmacologically acceptable salts thereof.

The clofibrate is represented by the following formula.

[Chemical Formula 10]

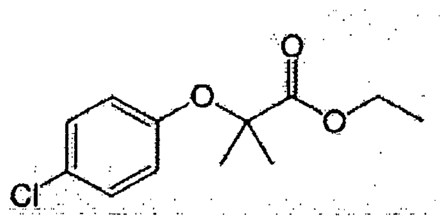

As used herein, the term "dopamine receptor antagonist" refers to a substance having a function to prevent dopamine from acting on receptors.
The dopamine receptor antagonist is not particularly limited, and examples thereof include a D1 receptor antagonist and a D5 receptor antagonist.
As used herein, the term "D1 receptor antagonist" refers to a substance having a function to prevent dopamine from acting on D1 receptors.
The D1 receptor antagonist is not particularly limited, and examples thereof include benzazepine, fenoldopam, lorcaserin, SCH23390, SCH39166, LE300, and derivatives thereof, as well as pharmacologically acceptable salts thereof.
The benzazepine is represented by the following formula.

[Chemical Formula 11]

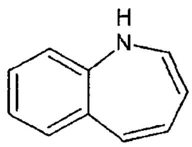

As used herein, the term "D5 receptor antagonist" refers to a substance having a function to prevent dopamine from acting on D5 receptors.
The D5 receptor antagonist is not particularly limited, and examples thereof include SCH39166 and derivatives thereof, as well as pharmacologically acceptable salts thereof.
The SCH39166 is represented by the following formula.

[Chemical Formula 12]

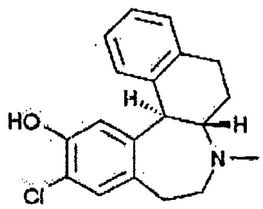

As used herein, the term "dopamine receptor agonist" refers to a substance having a function to act on dopamine receptors.
The dopamine receptor agonist is not particularly limited, and examples thereof include a D2 receptor agonist, a D3 receptor agonist, and a D4 receptor agonist.
As used herein, the term "D2 receptor agonist" refers to a substance having a function to act on D2 receptors.
The D2 receptor agonist is not particularly limited, and examples thereof include cabergoline, bromocriptine, pergolide, ropinirole, talipexole, aripiprazole, lurasidone, and derivatives thereof, as well as pharmacologically acceptable salts thereof.
The ropinirole is represented by the following formula.

[Chemical Formula 13]

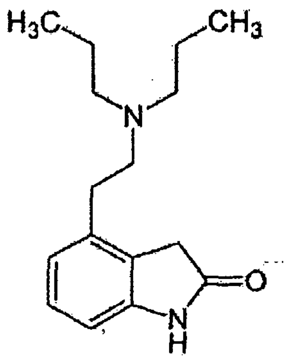

As used herein, the term "D3 receptor agonist" refers to a substance having a function to act on D3 receptors.

The D3 receptor agonist is not particularly limited, and examples thereof include piribedil, rotigotine, PD1289077, OH-DPAT, and derivatives thereof, as well as pharmacologically acceptable salts thereof.

The rotigotine is represented by the following formula.

[Chemical Formula 14]

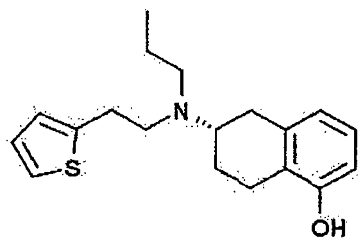

As used herein, the term "D4 receptor agonist" refers to a substance having a function to act on D4 receptors.

The D4 receptor agonist is not particularly limited, and examples thereof include flibanserin, ABT724, PD168077, CP226269, and derivatives thereof, as well as pharmacologically acceptable salts thereof.

The flibanserin is represented by the following formula.

[Chemical Formula 15]

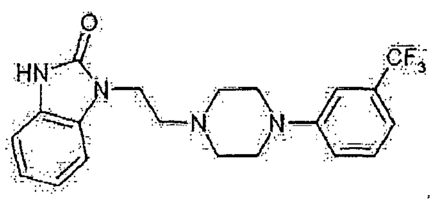

As used herein, the term "histamine receptor antagonist" refers to a substance having a function to prevent histamine from acting on receptors.

The histamine receptor antagonist is not particularly limited, and examples thereof include an H1 receptor antagonist and an H2 receptor antagonist.

As used herein, the term "H1 receptor antagonist" refers to a substance having a function to prevent histamine from acting on H1 receptors.

The H1 receptor antagonist is not particularly limited, and examples thereof include ketanserin, thonzylamine, mepyramine, tripelenamine, dimethindene, clemastine, bamipine, isothipendyl, chlorphenoxamine, dimetotiazine, chlorpromazine, hydroxyzine, opipramol, betahistine, cinnarizine, levocabastine, antazoline, diphenylpyraline, carbinoxamine, doxylamine, alimemazine, cyclizine, meclozine, levocetirizine, cyproheptadine, phenindamine, triprolidine, azatadine, astemizole, terfenadine, acrivastine, ebastine, desloratadine, rupatadine, bilastine, mizolastine, noberastine, rocastine, temelastine, bepotastine, diphenhydramin, chlorpheniramine, ketotifen, promethazine, cyproheptadine, epinastine, olopatadine, bepostadine, astemizole, emedastine, meguitazine, oxatomide, loratadine, fexofenadine, cetirizine,

azelastine, and derivatives thereof, as well as pharmacologically acceptable salts thereof.

The diphenhydramin is represented by the following formula.

[Chemical Formula 16]

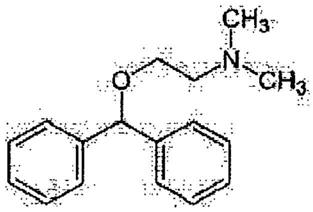

As used herein, the term "H2 receptor antagonist" refers to a substance having a function to prevent histamine from acting on H2 receptors.

The H2 receptor antagonist is not particularly limited, and examples thereof include cimetidine, ranitidine, famotidine, nizatidine, roxatidine, lafutidine, and derivatives thereof, as well as pharmacologically acceptable salts thereof.

The famotidine is represented by the following formula.

[Chemical Formula 17]

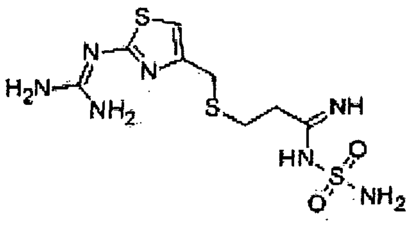

As used herein, the term "histamine receptor agonist" refers to a substance having a function to act on histamine receptors.

The histamine receptor agonist is not particularly limited, and examples thereof include an H1 receptor agonist, an H3 receptor agonist, and an H4 receptor agonist.

As used herein, the term "H1 receptor agonist" refers to a substance having a function to act on H1 receptors.

The H1 receptor agonist is not particularly limited, and examples thereof include 2-pyridylethylamine, 2-thiazolylethylamine, and derivatives thereof, as well as pharmacologically acceptable salts thereof.

The 2-pyridylethylamine is represented by the following formula.

[Chemical Formula 18]

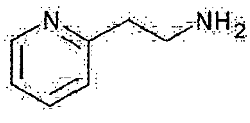

As used herein, the term "H3 receptor agonist" refers to a substance having a function to act on H3 receptors.

The H3 receptor agonist is not particularly limited, and examples thereof include immethridine, imetit, immepip, $\alpha$-methylhistamine, proxyfan, and derivatives thereof, as well as pharmacologically acceptable salts thereof.

The proxyfan is represented by the following formula.

[Chemical Formula 19]

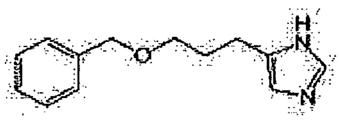

As used herein, the term "H4 receptor agonist" refers to a substance having a function to act on H4 receptors.

The H4 receptor agonist is not particularly limited, and examples thereof include 4-methylhistamine, VUF8430, immepip, and derivatives thereof, as well as pharmacologically acceptable salts thereof.

The 4-methylhistamine is represented by the following formula.

[Chemical Formula 20]

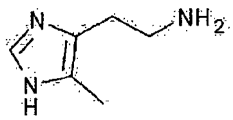

As used herein, the term "serotonin receptor antagonist" refers to a substance having a function to prevent serotonin from acting on receptors.

The serotonin receptor antagonist is not particularly limited, and examples thereof include 5-HT2 receptor antagonist, 5-HT4 receptor antagonist, 5-HT6 receptor antagonist, and 5-HT7 receptor antagonist.

As used herein, the term "5-HT2 receptor antagonist" refers to a substance having a function to prevent serotonin from acting on 5-HT2 receptors.

The 5-HT2 receptor antagonist is not particularly limited, and examples thereof include pizotifen, risperidone, olanzapine, quetiapine, aripiprazole, blonanserin, clozapine, paliperidone, ritanserin, yohimbine, mesulergine, agomelatine, cyclobenzaprine, sarpogrelate, methysergide, ketanserin, and derivatives thereof, as well as pharmacologically acceptable salts thereof.

The olanzapine is represented by the following formula:

[Chemical Formula 21]

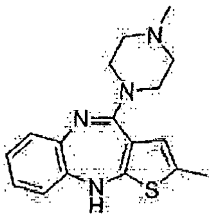

As used herein, the term "5-HT4 receptor antagonist" refers to a substance having a function to prevent serotonin from acting on 5-HT4 receptors.

The 5-HT4 receptor antagonist is not particularly limited, and examples thereof include piboserod, GR113808, GR125487, RS39604, SB204070, and derivatives thereof, as well as pharmacologically acceptable salts thereof.

The piboserod is represented by the following formula:

[Chemical Formula 22]

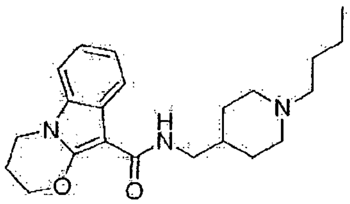

As used herein, the term "5-HT6 receptor antagonist" refers to a substance having a function to prevent serotonin from acting on 5-HT6 receptors.

The 5-HT6 receptor antagonist is not particularly limited, and examples thereof include cerlapirdine, clozapine, and derivatives thereof, as well as pharmacologically acceptable salts thereof.

The cerlapirdine is represented by the following formula:

[Chemical Formula 23]

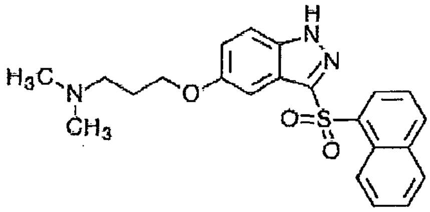

As used herein, the term "5-HT7 receptor antagonist" refers to a substance having a function to prevent serotonin from acting on 5-HT7 receptors.

The 5-HT7 receptor antagonist is not particularly limited, and examples thereof include metergoline and derivatives thereof, as well as pharmacologically acceptable salts thereof.

The metergoline is represented by the following formula.

[Chemical Formula 24]

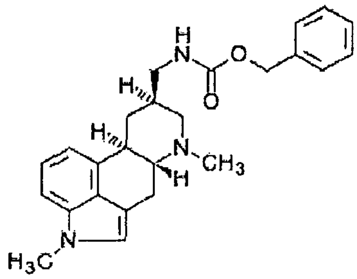

As used herein, the term "serotonin receptor agonist" refers to a substance having a function to act on serotonin receptors.

The serotonin receptor agonist is not particularly limited, and examples thereof include a 5-HT1 receptor agonist and a 5-HT2 receptor agonist.

As used herein, the term "5-HT1 receptor agonist" refers to a substance having a function to act on 5-HT1 receptors.

The 5-HT1 receptor agonist is not particularly limited, and examples thereof include piclozotan, tandospirone, sumatriptan, zolmitriptan, eletriptan, rizatriptan, naratriptan, almotriptan, frovatriptan, avitriptan, ergotamine, ergot alkaloid, and derivatives thereof, as well as pharmacologically acceptable salts thereof.

The zolmitriptan is represented by the following formula.

[Chemical Formula 25]

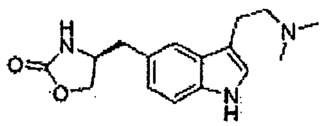

As used herein, the term "5-HT2 receptor agonist" refers to a substance having a function to act on 5-HT2 receptors.

The 5-HT2 receptor agonist is not particularly limited, and examples thereof include α-methyl-5-HT, agomelatine, norfenfluramine, metachlorophenylpiperazine, and derivatives thereof, as well as pharmacologically acceptable salts thereof.

The agomelatine is represented by the following formula.

[Chemical Formula 26]

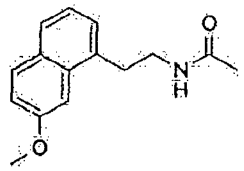

As used herein, the term "vasopressin receptor antagonist" refers to a substance having a function to prevent vasopressin from acting on receptors.

The vasopressin receptor antagonist is not particularly limited, and examples thereof include a V2 receptor antagonist.

As used herein, the term "V2 receptor antagonist" refers to a substance having a function to prevent vasopressin from acting on V2 receptors.

The V2 receptor antagonist is not particularly limited, and examples thereof include tolvaptan, mozavaptan, conivaptan, lixivaptan, and derivatives thereof, as well as pharmacologically acceptable salts thereof.

The mozavaptan is represented by the following formula.

[Chemical Formula 27]

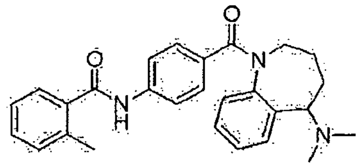

As used herein, the term "vasopressin receptor agonist" refers to a substance having a function to act on vasopressin receptors.

The vasopressin receptor agonist is not particularly limited, and examples thereof include a V1 receptor agonist.

As used herein, the term "V1 receptor agonist" refers to a substance having a function to act on V1 receptors.

The V1 receptor agonist is not particularly limited, and examples thereof include vasopressin, felypressin, desmopressin, lypressin, terlipressin, ornipressin, argipressin, and derivatives thereof, as well as pharmacologically acceptable salts thereof.

The desmopressin is represented by the following formula.

[Chemical Formula 28]

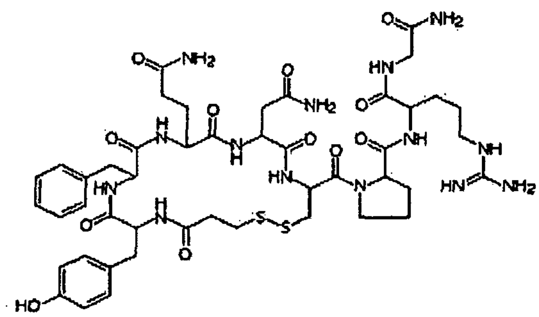

As used herein, the term "muscarinic receptor antagonist" refers to a substance having a function to act on muscarinic receptors.

The muscarinic receptor antagonist is not particularly limited, and examples thereof include an M1 receptor antagonist, an M3 receptor antagonist, and an M5 receptor antagonist.

As used herein, the term "M1 receptor antagonist" refers to a substance having a function to prevent acetylcholine from acting on M1 receptors. The term "M3 receptor antagonist" refers to a substance having a function to prevent acetylcholine from acting on M3 receptors. The term "M5 receptor antagonist" refers to a substance having a function to prevent acetylcholine from acting on M5 receptors.

The M1 receptor antagonist and/or the M3 receptor antagonist and/or the M5 receptor antagonist is not particularly limited, and examples thereof include pirenzepine, atropine, trimebutine, piperidolate, oxybutynin, tropicamide, propiverine, tolterodine, solifenacin, darifenacin, imidafenacin, oxyphencyclimine, tiotropium bromide, esoxybutynin, tiquizium, and derivatives thereof, as well as pharmacologically acceptable salts thereof.

The oxybutynin is represented by the following formula.

[Chemical Formula 29]

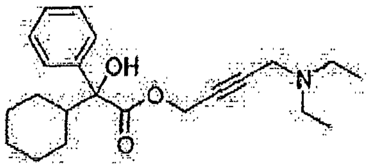

As used herein, the term "muscarinic receptor agonist" refers to a substance having a function to act on muscarine from acting on muscarinic receptors.

The muscarinic receptor agonist is not particularly limited, and examples thereof include an M1 receptor agonist, an M2 receptor agonist, an M3 receptor agonist, an M4 receptor agonist, and an M5 receptor agonist.

As used herein, the term "M1 receptor agonist" refers to a substance having a function to act on M1 receptors. The term "M2 receptor agonist" refers to a substance having a function to act on M2 receptors. The term "M3 receptor agonist" refers to a substance having a function to act on M3 receptors. The term "M4 receptor agonist" refers to a substance having a function to act on M4 receptors. The term "M5 receptor agonist" refers to a substance having a function to act on M5 receptors. The M1 receptor agonist and/or the M2 receptor agonist and/or the M3 receptor agonist and/or the M4 receptor agonist and/or the M5 receptor agonist is not particularly limited, and examples thereof include acetylcholine, aceclidine, alvameline, talsaclidine, xanomeline, pilocarpine, cevimeline, bethanechol, mazaticol, muscarine, and derivatives thereof, as well as pharmacologically acceptable salts thereof.

The bethanechol is represented by the following formula.

[Chemical Formula 30]

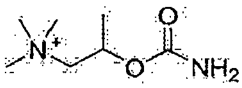

As used herein, the term "adrenergic receptor antagonist" refers to a substance having a function to prevent adrenaline from acting on receptors.

The adrenergic receptor antagonist is not particularly limited, and examples thereof include α1 receptor antagonist, β1 receptor antagonist, β2 receptor antagonist, and β3 receptor antagonist.

As used herein, the term "α1 receptor antagonist" refers to a substance having a function to prevent adrenaline from acting on α1 receptors.

The α1 receptor antagonist is not particularly limited, and examples thereof include prazosin, doxazosin, bunazosin, trimazosin, alfuzosin, silodosin, terazosin, tamusulosin, and derivatives thereof, as well as pharmacologically acceptable salts thereof.

The tamusulosin is represented by the following formula.

[Chemical Formula 31]

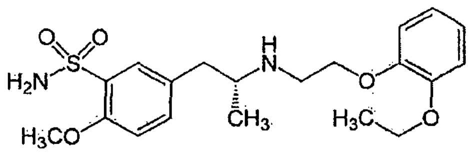

As used herein, the term "β1 receptor antagonist" refers to a substance having a function to prevent adrenaline from acting on β1 receptors. The term "β2 receptor antagonist" refers to a substance having a function to prevent adrenaline from acting on β2 receptors. The term "β3 receptor antagonist" refers to a substance having a function to prevent adrenaline from acting on β3 receptors.

The β1 receptor antagonist and/or the β2 receptor antagonist and/or the β3 receptor antagonist is not particularly limited, and examples thereof include bopindolol, pindolol, timolol, dichloroisoprenaline, alprenolol, carteolol, indenolol, bunitrolol, penbutolol, propranolol, nadolol, nipradilol, tilisolol, acebutolol, celiprolol, metoprolol, atenolol, bisoprolol, betaxolol, practolol, bevantolol, butoxamine, carvedilol, amosulalol, arotinolol, labetalol, and derivatives thereof, as well as pharmacologically acceptable salts thereof.

The propranolol is represented by the following formula.

[Chemical Formula 32]

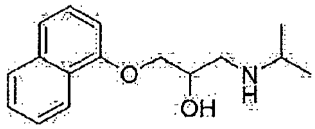

As used herein, the term "adrenergic receptor agonist" refers to a substance having a function to act on adrenergic receptors.

The adrenergic receptor agonist is not particularly limited, and examples thereof include α1 receptor agonist and α2 receptor agonist.

As used herein, the term "α1 receptor agonist" refers to a substance having a function to act on α1 receptors. The term "α2 receptor agonist" refers to a substance having a function to act on α2 receptors.

The α1 receptor agonist and/or α2 receptor agonist is not particularly limited, and examples thereof include norepinephrine, norfenefrine, etilefrine, naphazoline, phenylephrine, midodrine, methoxamine, oxedrine, metaraminol, arbutamine, ephedrine, oxymetazoline, tetryzoline, xylometazoline, tramazoline, pseudoephedrine, dipivefrine, amidephrine, methylephedrine, rilmenidine, brimonidine, medetomidine, xylazine, tizanidine, guanfacine, methyldopa, guanabenz, and derivatives thereof, as well as pharmacologically acceptable salts thereof.

The xylazine is represented by the following formula.

[Chemical Formula 33]

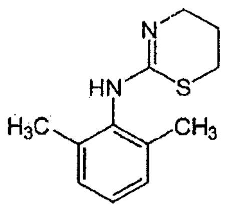

As used herein, the term "angiotensin receptor agonist" refers to a substance having a function to act on angiotensin receptors.

The angiotensin receptor agonist is not particularly limited, and examples thereof include AT2 receptor agonist.

As used herein, the term "AT2 receptor agonist" refers to a substance having a function to act on AT2 receptors.

The AT2 receptor agonist is not particularly limited, and examples thereof include novokinin, angiotensin, and derivatives thereof, as well as pharmacologically acceptable salts thereof.

The angiotensin is represented by the following formula.

[Chemical Formula 34]

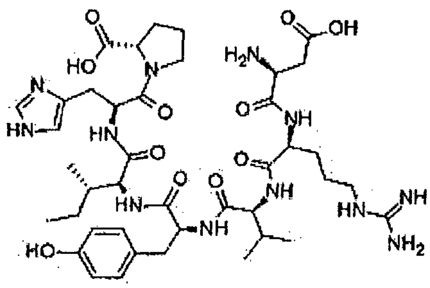

As used herein, the term "GABA receptor agonist" refers to a substance having a function to act on GABA receptors.

The GABA receptor agonist is not particularly limited, and examples thereof include $GABA_B$ receptor agonist.

As used herein, the term "$GABA_B$ receptor agonist" refers to a substance having a function to act on $GABA_B$ receptors.

The $GABA_B$ receptor agonist is not particularly limited, and examples thereof include baclofen, γ-aminobutyric acid, arbaclofen, and derivatives thereof, as well as pharmacologically acceptable salts thereof.

The baclofen is represented by the following formula.

[Chemical Formula 35]

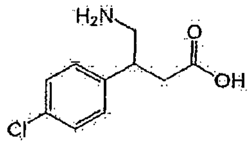

As used herein, the term "thrombin receptor antagonist" refers to a substance having a function to prevent thrombin from acting on receptors.

The thrombin receptor antagonist is not particularly limited, and examples thereof include PAR-1 receptor antagonist.

As used herein, the term "PAR-1 receptor antagonist" refers to a substance having a function to prevent thrombin from acting on PAR-1 receptors.

The PAR-1 receptor antagonist is not particularly limited, and examples thereof include vorapaxar, atopaxar, FR171113, RWJ56110, dabigatran, dabigatran etexilate, melagatran, ximelagatran, hirudin, hirulog, argatroban, and derivatives thereof, as well as pharmacologically acceptable salts thereof.

The vorapaxar is represented by the following formula.

[Chemical Formula 36]

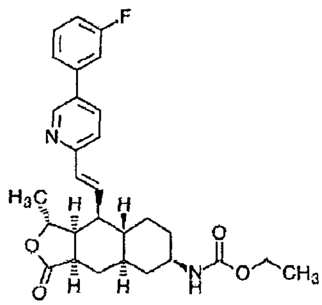

As used herein, the term "thrombin receptor agonist" refers to a substance having a function to act on thrombin receptors.

The thrombin receptor agonist is not particularly limited, and examples thereof include PAR-1 receptor agonist.

As used herein, the term "PAR-1 receptor agonist" refers to a substance having a function to act on PAR-1 receptors.

The PAR-1 receptor agonist is not particularly limited, and examples thereof include TRAP-6, TRAP-14, NAT6-$NH_2$, and derivatives thereof, as well as pharmacologically acceptable salts thereof.

The TRAP-6 is represented by the following formula.

[Chemical Formula 37]

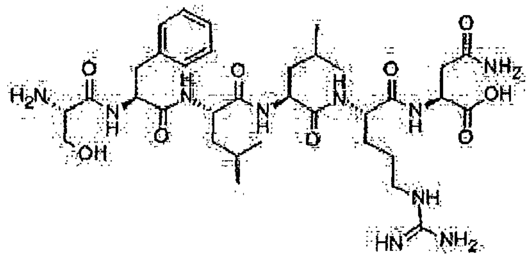

As used herein, the term "opioid receptor agonist" refers to a substance having a function to act on opioid receptors.

The opioid receptor agonist is not particularly limited, and examples thereof include trimebutine, alvimopan, morphine, oxycodone, dihydrocodeine, diamorphine, pethidine, pentazocine, buprenorphine, butorphanol, nalbuphine, tilidine, dezocine, meptazinol, tapentadol, naltrexone, methadone, ethylmorphine, hydrocodone, acetyl dihydrocodeine, nalorphine, naloxone, remoxipride, opipramol, and derivatives thereof, as well as pharmacologically acceptable salts thereof.

The buprenorphine is represented by the following formula.

[Chemical Formula 38]

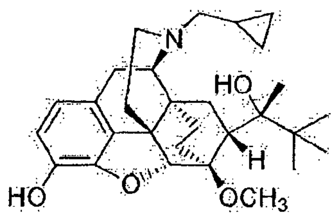

As used herein, the term "leukotriene receptor antagonist" refers to a substance having a function to prevent leukotriene from acting on receptors.

The leukotriene receptor antagonist is not particularly limited, and examples thereof include CysLT1 receptor antagonist and CysLT2 receptor antagonist.

As used herein, the term "CysLT1 receptor antagonist" refers to a substance having a function to prevent leukotriene from acting on CysLT1 receptors. The term "CysLT2 receptor antagonist" refers to a substance having a function to prevent leukotriene from acting on CysLT2 receptors.

The CysLT1 receptor antagonist and/or CysLT2 receptor antagonist is not particularly limited, and examples thereof include montelukast, zafirlukast, pranlukast, and derivatives thereof, as well as pharmacologically acceptable salts thereof.

Examples of pharmacologically acceptable salts of the montelukast include montelukast sodium.

The montelukast sodium is represented by the following formula.

[Chemical Formula 39]

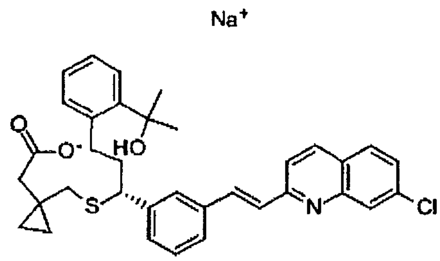

As used herein, the term "leukotriene receptor agonist" refers to a substance having a function to act on leukotriene receptors.

The leukotriene receptor agonist is not particularly limited, and examples thereof include BLT receptor agonist.

As used herein, the term "BLT receptor agonist" refers to a substance having a function to act on BLT receptors.

The BLT receptor agonist is not particularly limited, and examples thereof include leukotriene B4, CAY10583, and derivatives thereof, as well as pharmacologically acceptable salts thereof.

The leukotriene B4 is represented by the following formula.

[Chemical Formula 40]

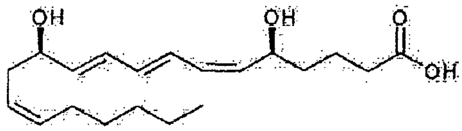

As used herein, the term "ADP receptor agonist" refers to a substance having a function to act on ADP receptors.

The ADP receptor agonist is not particularly limited, and examples thereof include adenosine diphosphate and derivatives thereof, as well as pharmacologically acceptable salts thereof.

The adenosine diphosphate is represented by the following formula.

[Chemical Formula 41]

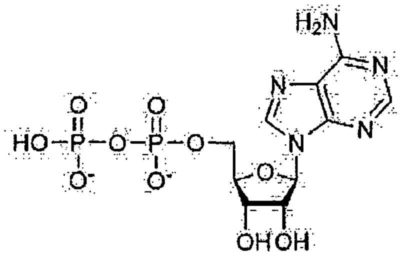

As used herein, the term "melatonin receptor agonist" refers to a substance having a function to act on melatonin receptors.

The melatonin receptor agonist is not particularly limited, and examples thereof include melatonin, perlapine, tasimelteon, and derivatives thereof, as well as pharmacologically acceptable salts thereof.

The melatonin is represented by the following formula.

[Chemical Formula 42]

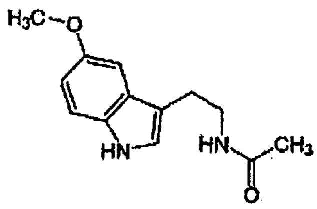

As used herein, the term "somatostatin receptor agonist" refers to a substance having a function to act on melatonin receptors.

The somatostatin receptor agonist is not particularly limited, and examples thereof include somatostatin, octreotide, and derivatives thereof, as well as pharmacologically acceptable salts thereof.

The octreotide is represented by the following formula.

[Chemical Formula 43]

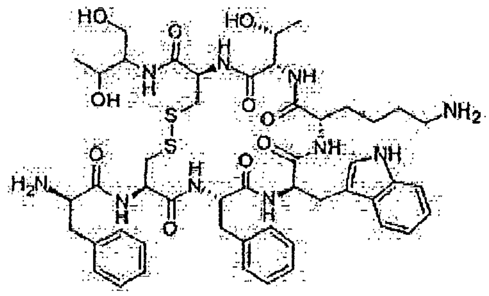

As used herein, the term "cannabinoid receptor agonist" refers to a substance having a function to act on cannabinoid receptors.

The cannabinoid receptor agonist is not particularly limited, and examples thereof include dronabinol, nabilone, levonantradol, otenabant, GW833972A, GW405833, and derivatives thereof, as well as pharmacologically acceptable salts thereof.

The dronabinol is represented by the following formula.

[Chemical Formula 44]

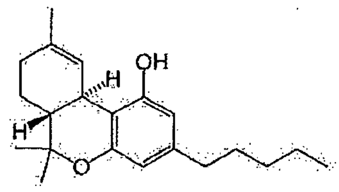

As used herein, the term "sphingosine-1 phosphate receptor agonist" refers to a substance having a function to act on sphingosine-1 phosphate receptors.

The sphingosine-1 phosphate receptor agonist is not particularly limited, and examples thereof include fingolimod and derivatives thereof, as well as pharmacologically acceptable salts thereof.

The fingolimod is represented by the following formula.

[Chemical Formula 45]

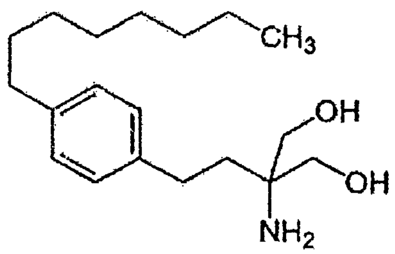

As used herein, the term "metabotropic glutamate receptor agonist" refers to a substance having a function to act on metabotropic glutamate receptors.

The metabotropic glutamate receptor agonist is not particularly limited, and examples thereof include mGluR2 receptor agonist, mGluR3 receptor agonist, mGluR4 receptor agonist, mGluR6 receptor agonist, mGluR7 receptor agonist, and mGluR8 receptor agonist.

As used herein, the term "mGluR2 receptor agonist" refers to a substance having a function to act on mGluR2 receptors. The term "mGluR3 receptor agonist" refers to a substance having a function to act on mGluR3 receptors. The term "mGluR4 receptor agonist" refers to a substance having a function to act on mGluR4 receptors. The term "mGluR6 receptor agonist" refers to a substance having a function to act on mGluR6 receptors. The term "mGluR7

receptor agonist" refers to a substance having a function to act on mGluR7 receptors. The term "mGluR8 receptor agonist" refers to a substance having a function to act on mGluR8 receptors.

The mGluR2 receptor agonist and/or the mGluR3 receptor agonist and/or the mGluR4 receptor agonist and/or the mGluR6 receptor agonist and/or the mGluR7 receptor agonist and/or the mGluR8 receptor agonist is not particularly limited, and examples thereof include VU0361737, VU0155041, Biphenylindanone A, PBDA, L-AP4, and derivatives thereof, as well as pharmacologically acceptable salts thereof.

The VU0361737 is represented by the following formula.

[Chemical Formula 46]

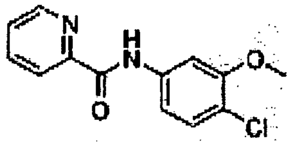

As used herein, the term "phospholipase A2 inhibitor" refers to a substance having a function to suppress the activity of phospholipase A2.

The phospholipase A2 inhibitor is not particularly limited, and examples thereof include glycyrrhizic acid, glycyrrhetic acid, and derivatives thereof, as well as pharmacologically acceptable salts thereof.

The glycyrrhetic acid is represented by the following formula.

[Chemical Formula 47]

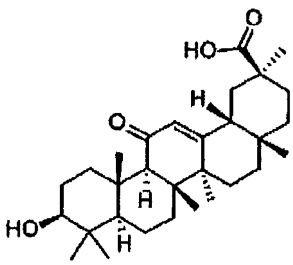

As used herein, the term "TGF-β production inhibitor" refers to a substance having a function to suppress the production of TGF-β.

The TGF-β production inhibitor is not particularly limited, and examples thereof include pirfenidone, tranilast, and derivatives thereof, as well as pharmacologically acceptable salts thereof.

The pirfenidone is represented by the following formula.

[Chemical Formula 48]

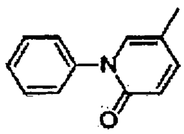

As used herein, the term "Th2 cytokine inhibitor" refers to a substance having a function to suppress the production of Th2 cytokines such as IL-4 and IL-5.

The Th2 cytokine inhibitor is not particularly limited, and examples thereof include suplatast and derivatives thereof, as well as pharmacologically acceptable salts thereof.

Pharmacologically acceptable salts of the suplatast include suplatast tosylate. The Th2 cytokine inhibitor is preferably suplatast tosylate.

The suplatast tosylate is represented by the following formula.

[Chemical Formula 49]

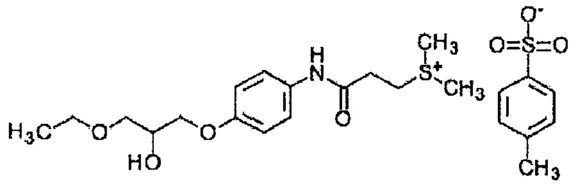

As used herein, the term "TLR ligand" refers to a ligand of Toll-like receptor (TLR).

The TLR ligand is not particularly limited, and examples thereof include ligand of TLRs 1 to 9. Specifically, examples thereof include TLR1/2 ligand, TLR2/6 ligand, TLR2 and Dectin-1 ligand, TLR3 ligand, TLR4 ligand, TLR5 ligand, TLR7 and/or TLR8 ligand, and TLR9 ligand. In particular, the TLR ligand is preferably TLR1/2 ligand, TLR2 and Dectin-1 ligand, TLR3 ligand, TLR4 ligand, TLR7 and/or TLR8 ligand, and/or TLR9 ligand.

As used herein, the term "TLR1/2 ligand" refers to a ligand of a heterodimer of Toll-like receptor (TLR) 1 and Toll-like receptor (TLR) 2. The TLR1/2 ligand is not particularly limited, and examples thereof include a triacylated lipoprotein derived from bacterial cell walls and a salt thereof. Any of these may be an extract, a product, or a synthetic product. In particular, $Pam_3CSK_4$ represented by the following formula is preferred.

[Chemical Formula 50]

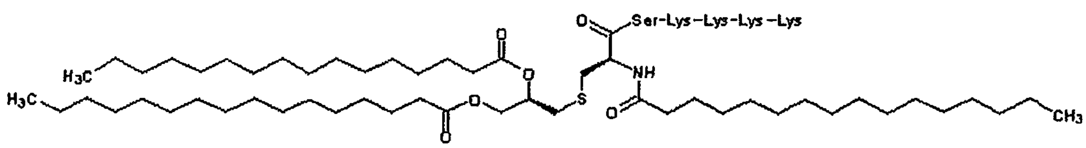

As used herein, the term "TLR2 and Dectin-1 ligand" refers to a ligand for Toll-like receptor (TLR) 2 and β1,3-glucan receptor (Dectin-1).

The TLR2 and Dectin-1 ligand is not particularly limited, and examples thereof include β1,3-glucan derived from fungal cell walls, and salts thereof. Any of these may be an extract, a product, or a synthetic product. Zymosan derived from yeast cell walls is particularly preferred.

As used herein, the term "TLR3 ligand" refers to a ligand for Toll-like receptor (TLR) 3.

The TLR3 ligand is not particularly limited, and examples thereof include a double-stranded RNA (dsRNA) derived from a virus and a salt thereof. Any of these may be an extract, a product, or a synthetic product. In particular, the TLR3 ligand is preferably polyinosinic-polycytidylic acid (Poly(I:C), which is a synthetic product, and/or a salt thereof.

As used herein, the term "TLR7 and/or TLR8 ligand" refers to a ligand of Toll-like receptor (TLR) 7 and/or TLR8.

The TLR7 and/or TLR8 ligand is not particularly limited, and examples thereof include single-stranded RNA, imiquimod, resiquimod (R848), TLR7-II, loxoribine, and bropirimine. In particular, imiquimod, resiquimod, and TLR7-II, bropirimine are preferred.

The imiquimod is 1-(2-methylpropyl)-1H-imidazo[4,5-c]quinoline-4-amine represented by the following formula. Its characteristics and production method are described in JP-T H07-505883, for example.

[Chemical Formula 51]

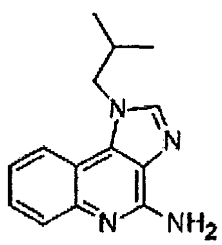

The resiquimod is 4-amino-2-(ethoxymethyl)-α,α-dimethyl-1H-imidazo[4,5-c]quin oline-1-ethanol represented by the following formula.

[Chemical Formula 52]

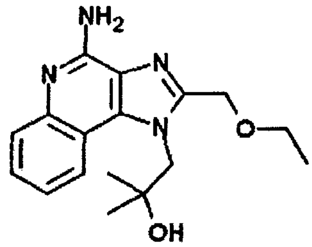

The TLR7-II is represented by the following formula.

[Chemical Formula 53]

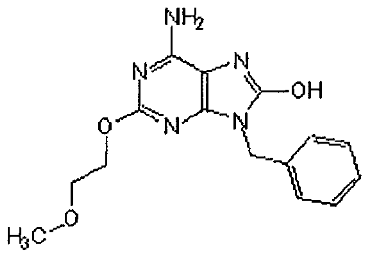

The bropirimine is represented by the following formula.

[Chemical Formula 54]

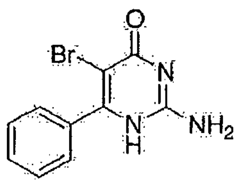

As used herein, the term "TLR9 ligand" refers to a ligand of Toll-like receptor (TLR) 9.

The TLR9 ligand is not particularly limited, but ODN 1826 is preferred. Any of these may be an extract, a product, or a synthetic product. The ODN 1826 is an oligodeoxynucleotide having the following sequence (SEQ ID No: 12).

5' - t c c a t g a c g t t c c t g a c g t - 3'

As used herein, the term "TLR2/6 ligand" refers to a ligand of a heterodimer of Toll-like receptor (TLR) 2 and Toll-like receptor (TLR) 6.

The TLR2/6 ligand is not particularly limited, and examples thereof include a diacylated lipoprotein derived from mycoplasma cell walls and a salt thereof. Any of these may be an extract, a product, or a synthetic product. In particular, $Pam_2CSK_4$, MALP-2, and FSL-1 are preferred.

The $Pam_2CSK_4$ is represented by the following formula.

[Chemical Formula 55]

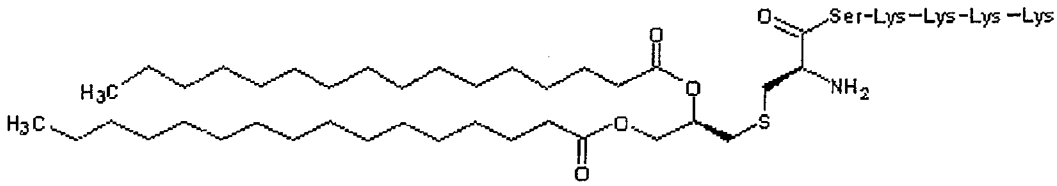

The FSL-1 is represented by the following formula.

[Chemical Formula 56]

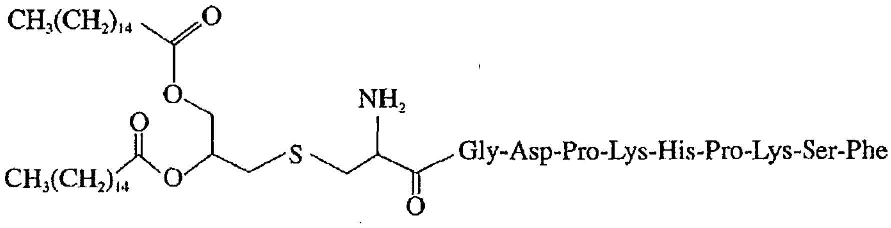

As used herein, the term "TLR5 ligand" refers to a ligand of Toll-like receptor (TLR) 5.

The TLR5 ligand is not particularly limited, and examples thereof include flagellin. Any of these may be an extract, a product, or a synthetic product.

The Toll-like receptor (TLR) is a family of type I transmembrane proteins. The Toll-like receptor initiates, by in vivo activation thereof, congenital immune response in which a specific cytokine, a specific chemokine, and a specific growth factor are involved. While all the TLRs can activate certain intracellular signal transmission molecules (such as a nuclear factor κB (NF-κB) and mitogen-activated protein kinase (MAP kinase)), a specific population of a cytokine and a chemokine which are released seems to be inherent to each TLR. TLR3, 7, 8, and 9 include a subfamily of TLR which is present in an endsome fraction or a lysosome fraction of an immune cell (e.g., dendritic cell or monocyte). Specifically, TLR3 is expressed by a wide range of cells such as dendritic cells and fibroblasts. TLR7 is expressed by plasma cell-like dendritic cells and is also expressed by monocytes to a lesser extent. TLR8 is expressed by monocytes, monocyte-derived dendritic cells, and myeloid dendritic cells. TLR9 is expressed by plasma cell-like dendritic cells. This subfamily mediates recognition of a microorganism nucleic acid (single-stranded RNA, double-stranded RNA, single-stranded DNA, or the like). TLR3, TLR7 and/or TLR8, and TLR9 agonists stimulate production of various inflammatory cytokines (e.g., interleukin-6, interleukin-12, TNF-α, and interferon-y). Such agonists also promote an increase in expression of costimulatory molecules (e.g., CD40, CD80, and CD86), major histocompatibility complex molecules, and chemokine receptors. Type I interferons (IFNα and IFNβ) are also produced by cells upon activation with TLR7 and/or TLR8 agonist.

As used herein, the term "cyclic dinucleotide" refers to a molecule in which two OH groups of a sugar moiety of two nucleotides produce an ester for each same phosphoric acid molecule so that nucleotides are cyclized, and an analog thereof.

The cyclic dinucleotide is not particularly limited, and examples thereof include cyclic diAMP (c-di-AMP), cyclic diGMP (c-di-GMP), c-dGpGp, c-dGpdGp, c-GpAp, c-GpCp, and c-GpUp. In particular, the cyclic dinucleotide is preferably cyclic diGMP and/or cyclic diAMP.

The cyclic dinucleotide activates dendritic cells or T cells and is known to be used as an adjuvant. Its characteristics and production method are described in JP-T 2007-529531 (Patent Literature 5), for example.

The cyclic diGMP has the following formula, and a synthesis method thereof is described in Kawai et al., Nucleic Acids Research Suppl. 3:103-4.

[Chemical Formula 57]

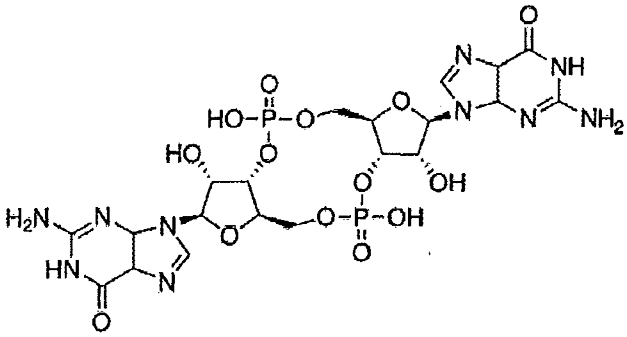

As used herein, the term "immunomodulatory small molecule drug" refers to a substance that activates or suppresses immune cells such as T cells, NK cells, and macrophages, and that does not correspond to any of the above-mentioned TLR ligands, cyclic dinucleotides, helper peptides, cyclooxygenase inhibitors, prostaglandin receptor antagonists, prostaglandin receptor agonists, TSLP production inhibitors, adenylate cyclase inhibitors, omega-3 fatty acids, PPAR agonists, dopamine receptor antagonists, dopamine receptor agonists, histamine receptor agonists, histamine receptor antagonists, serotonin receptor agonists, serotonin receptor antagonists, vasopressin receptor antagonists, vasopressin receptor agonists, muscarinic receptor antagonists, muscarinic receptor agonist, adrenergic receptor antagonists, adrenergic receptor agonists, angiotensin receptor agonists, GABA receptor agonists, thrombin receptor antagonists, thrombin receptor agonists, opioid receptor agonists, ADP receptor agonists, leukotriene receptor antagonists, leukotriene receptor agonists, melatonin receptor agonist, somatostatin receptor agonist, cannabinoid receptor agonist, sphingosine-1 phosphate receptor agonists, metabotropic glutamate receptor agonists, phospholipase A2 inhibitors, TGF-β production inhibitors, or Th2 cytokine inhibitors.

The immunomodulatory small molecule drug is not particularly limited, and examples thereof include bestatin, pidotimod, levamisole, golotimod, forphenicinol, and derivatives thereof, as well as pharmacologically acceptable salts thereof. Specifically, pharmacologically acceptable salts of levamisole include levamisole hydrochloride.

The bestatin is represented by the following formula.

[Chemical Formula 58]

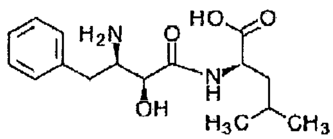

The pidotimod is represented by the following formula.

[Chemical Formula 59]

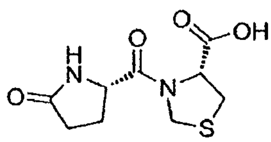

The levamisole hydrochloride is represented by the following formula.

[Chemical Formula 60]

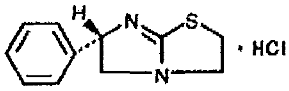

In the present invention, the immunomodulatory small molecule drug is a compound usually having a molecular weight of less than 1000, preferably less than 500. In a preferred embodiment of the present invention, the immunomodulatory small molecule drug is at least one compound selected from the group consisting of bestatin, pidotimod, and levamisole hydrochloride.

The vaccine pharmaceutical composition of the present invention preferably induces cellular immunity by multiple dose administration.

The cellular immunity inducing effect of the vaccine pharmaceutical composition of the present invention from the second and subsequent administrations is preferably 100% or higher, as compared to the cellular immunity inducing effect of the vaccine pharmaceutical composition from the preceding administration.

The cellular immunity inducing effect can be measured by an immunity induction experiment using an animal model for immunological evaluation and also by ELISPOT assay (IFN-$\gamma$). A spleen in an animal model for immunological evaluation is used as a sample for measurement of the cellular immunity.

The vaccine pharmaceutical composition of the present invention preferably contains the first cellular immunity induction promoter in a weight ratio of 150 parts by weight or more per part by weight of the antigen in the composition.

Administration of the antigen and the first cellular immunity induction promoter at the above ratio can suppress, with a suitable percentage, induction of apoptosis of antigen-specific CD8-positive T cells, and can effectively induce cellular immunity.

As used herein, the "weight ratio of the first cellular immunity induction promoter administered to the antigen administered" refers to the weight ratio of the first cellular immunity induction promoter to the antigen, which are actually administered to the body of an animal model for immunological evaluation.

With respect to the vaccine pharmaceutical composition for subcutaneous, intradermal, or intramuscular injection, the weight ratio of the first cellular immunity induction promoter administered to the antigen administered is the same as that in the dose injected to an immunized mouse. In other words, the weight ratio is the same as that of the first cellular immunity induction promoter to the antigen in the vaccine pharmaceutical composition of the present invention.

In contrast, with respect of the vaccine pharmaceutical composition for transdermal or transmucosal administration, the antigen actually administered and the first cellular immunity induction promoter actually administered are affected by skin or mucosal resistance to diffusion, and thus the doses of these are not always the same as those transdermally or transmucosally administered to an immunized mouse. The skin or mucosal resistance to diffusion is affected by the molecular weight, hydrophilic degree, and hydrophobic degree of the antigen and the first cellular immunity induction promoter. Thus, the weight ratio of the first cellular immunity induction promoter to the antigen contained in the vaccine pharmaceutical composition of the present invention can be controlled by increasing or decreasing the antigen and/or the first cellular immunity induction promoter in response to the skin or mucosal resistance to diffusion of the antigen and the first cellular immunity induction promoter.

Specifically, with respect to the vaccine pharmaceutical composition for transdermal administration, the weight ratio of the first cellular immunity induction promoter is adjusted to 0.0001 to 1000 parts by weight per part by weight of the antigen in the vaccine pharmaceutical composition. In this manner, the weight ratio of the cellular immunity induction promoter actually administered can be adjusted to 150 parts by weight or more per part by weight of the antigen actually administered. In terms of stimulation of the antigen and the first cellular immunity induction promoter, the amount of the first cellular immunity induction promoter administered is preferably 3000 parts by weight or less per part by weight of the antigen administered.

Preferably, the vaccine pharmaceutical composition of the present invention further contains a helper peptide as a second cellular immunity induction promoter.

Use of the second cellular immunity induction promoter (i.e., helper peptide) in combination with the first cellular immunity induction promoter can further promote cellular immunity.

As used herein, the term "helper peptide" refers to any peptide that activates helper T cells.

The helper peptide is not particularly limited, and examples thereof include a helper peptide derived from tubercle bacillus, a helper peptide derived from measles virus, a helper peptide derived from hepatitis B virus, a helper peptide derived from hepatitis C virus, a helper peptide derived from Chlamydia trachomatis, a helper peptide derived from P. falciparum sporozoite, a helper peptide derived from keyhole limpet haemocyanin, a helper peptide derived from tetanus toxin, a helper peptide derived from pertussis toxin, a helper peptide derived from diphtheria toxin, helper peptides derived from cancer cells (e.g., IMA-MMP-001 helper peptide, CEA-006 helper peptide, MMP-001 helper peptide, TGFBI-004 helper peptide, HER-2/neu(aa776-790) helper peptide, AE36 helper peptide, AE37 helper peptide, MET-005 helper

peptide, and BIR-002 helper peptide), and universal helper analogs (e.g., PADRE). In particular, Peptide-25, altered Peptide-25, and PADRE are preferred. Examples of the altered Peptide-25 include Peptide-25B.

As used herein, the term "Peptide-25" refers to a peptide of 15 amino acids having a sequence of Phe Gln Asp Ala Tyr Asn Ala Ala Gly Gly His Asn Ala Val Phe (SEQ ID No: 13). The sequence corresponds to the amino acid residues 240 to 254 of Ag85B which is a major protein secreted by Mycobacterium tuberculosis.

As used herein, the term "Peptide-25B" refers to a peptide of 15 amino acids prepared from Peptide-25 by altering a part of its amino acids to enhance the immunomodulatory effect, having a sequence of Phe Gln Asp Ala Tyr Asn Ala Val His Ala Ala His Ala Val Phe (SEQ ID No: 14).

As used herein, the term "PADRE" refers to a peptide of 13 amino acids having a sequence of D-Ala Lys cyclohexyl-Ala Val Ala Ala Trp Thr Leu Lys Ala Ala D-Ala (SEQ ID No: 15).

In addition, a peptide obtained by altering all or a part of amino acids of the peptide described above by, for example, substitution or modification (hereinafter also referred to as "altered helper peptide") can also be used instead of or in combination with the helper peptide described above.

Examples of altered helper peptides include a peptide having an amino acid sequence obtained by substituting, deleting, or adding one to several amino acids (for example, 1, 2, 3, 4, or 5 amino acids) in an amino acid sequence of the original helper peptide, and a peptide having an amino acid sequence obtained by modifying one to several amino acids (for example, 1, 2, 3, 4, 5, 6, 7, 8, 9, 10, 11, 12, 13, 14, or 15 amino acids) in an amino acid sequence of the original helper peptide.

The amino acids of the altered helper peptide may be modified in any manner. Examples of such modifications include acetylation; alkylation such as methylation; glycosylation; hydroxylation; carboxylation; aldehydation; phosphorylation; sulfonylation; formylation; aliphatic chain addition modification such as myristoylation, palmitoylation, and stearoylation; octanoylation; esterification; amidation; deamidation; disulfide bond formation modification such as cystine modification, glutathione modification, and thioglycolic acid modification; glycation; ubiquitination; succinimide formation; glutamylation; and prenylation. In the altered helper peptide, substitution, deletion, or addition of one or more amino acids may be combined with modification of one or more amino acids.

As used herein, the term "subject" refers to any animal in which immune response can be induced by administration of the vaccine pharmaceutical composition at the practical stage. Typically, the term refers to mammals including human, mouse, rat, canine, feline, leporine, equine, bovine, ovine, porcine, caprine, simian, and chimpanzee. A particularly preferred subject is a human.

As used herein, the term "animal model for immunological evaluation" refers to an animal model used for evaluation of immunity induction characteristics of the vaccine pharmaceutical composition. Specifically, the term refers to an animal model used for evaluation of the level of induction of cellular immunity. An animal used as the animal model for immunological evaluation is one which allows for evaluation of induction of cellular immunity by the antigen in the vaccine pharmaceutical composition, in view of compatibility between the antigen in the vaccine pharmaceutical composition and MHC class I molecules of the animal. For example, in the case of a vaccine pharmaceutical composition containing HLA-A*24 type MHC-restricted class I peptide, evaluation is carried out using BALB/c mice. In the case of a vaccine pharmaceutical composition containing HLA-A*02 type MHC-restricted peptide, evaluation is carried out using genetically engineered mice which allow for evaluation of induction of cellular immunity by HLA-A*02 type MHC-restricted peptide. In the case of a vaccine pharmaceutical composition containing another HLA type MHC-restricted peptide, evaluation is carried out using an animal which allows for evaluation of induction of cellular immunity by the HLA type MHC-restricted peptide. In the case of a vaccine pharmaceutical composition containing a protein antigen, evaluation is carried out using an animal having MHC compatible with a class I epitope as the target of cellular immunity induction, among various class 1 epitopes included in the amino acid sequence of the protein antigen. In the case where the animal is shaved to provide a site for transdermal administration, the animal should be used after it is fully recovered from skin damage caused by shaving.

As used herein, the "pharmacologically acceptable salt" that can be contained in the pharmaceutical composition of the present invention refers to a salt that does not adversely affect the subject to be administered and that does not eliminate the pharmacological activity of the components of the pharmaceutical composition. Non-limiting examples include inorganic acid salts (e.g., hydrochloride and phosphate), organic acid salts (e.g., acetate, phthalate, TFA salt), metal salts (alkali metal salts (e.g., sodium salt and potassium salt), alkaline-earth metal salts (e.g., calcium salt and magnesium salt), aluminium salt), and amine salts (e.g., triethylamine salt, benzylamine salt, diethanolamine salt, t-butylamine salt, dicyclohexylamine salt, arginine salt, dimethylammonium salt, and ammonium salt).

<Vaccine pharmaceutical composition for transdermal administration>

As used herein, the term "transdermal administration" as in the pharmaceutical composition for transdermal administration may refer to any formulation that is usually used for transdermal administration, for example, a solution for external application such as a liniment or a lotion; a spray for external application such as an aerosol; an ointment;

a plaster; a cream; a gel; or a patch such as a tape or a poultice. Categories, definitions, properties, production processes, and the like of these formulations are well known in the relevant art. For example, see the Japanese Pharmacopoeia, 16th Edition. Any known material may be used for these formulations.

The amount of the antigen in the vaccine pharmaceutical composition for transdermal administration of the present invention is not particularly limited, but the lower limit of the amount based on the total weight of the composition is preferably 0.001% by weight, more preferably 0.01% by weight, still more preferably 0.1% by weight, and the upper limit thereof is preferably 100% by weight, more preferably 70% by weight, still more preferably 40% by weight.

The amount of the first cellular immunity induction promoter in the vaccine pharmaceutical composition for transdermal administration of the present invention is not particularly limited, but the lower limit of the amount per part by weight of the antigen is preferably 0.0001 parts by weight, more preferably 0.001 parts by weight, still more preferably 0.01 parts by weight, and the upper limit thereof is preferably 1000 parts by weight, more preferably 100 parts by weight, still more preferably 10 parts by weight.

The amount of the second cellular immunity induction promoter in the vaccine pharmaceutical composition for transdermal administration of the present invention is not particularly limited, but the lower limit of the amount per part by weight of the antigen is preferably 0.0001 parts by weight, more preferably 0.001 parts by weight, still more preferably 0.01 parts by weight, and the upper limit thereof is preferably 1000 parts by weight, more preferably 100 parts by weight, still more preferably 10 parts by weight.

If the amount is less than the lower limit, the immunity inducing effect may be insufficient. If the amount is more than the upper limit, the safety may be compromised.

Any base may be used for the liniment. Examples thereof include water; ethanol; fatty oils such as hard paraffin, soft paraffin, liquid paraffin, glycerin, paraffin oil, beeswax, and metal soap; mucilage; natural oils (such as almond oil, corn oil, peanut oil, castor oil, olive oil, and derivatives thereof (e.g., polyoxyl castor oil)); mutton tallow or derivatives thereof; and fatty acids and/or esters (e.g., stearic acid, oleic acid, and isopropyl myristate).

The lotion is not particularly limited. For example, it is a formulation in which an active component is homogeneously dispersed in an aqueous solution, and there are a suspension-type lotion and an emulsion-type lotion. Examples of the suspending agent include gum arabic, sodium alginate, sodium carboxymethyl cellulose, methylcellulose, and bentonite. Examples of the emulsifier include sodium lauryl sulfate and sorbitan fatty acid esters.

Any base may be used for the ointment. For example, generally, oils/fats, waxes, and hydrocarbon compounds as hydrophobic bases can be used. Specific examples of the base for the ointment include mineral bases such as yellow Vaseline, white Vaseline, paraffin, liquid paraffin, plastibase, and silicone, and animal or plant bases such as beeswax and animal or plant fats and/or oils.

Any base may be used for the cream. Examples thereof include water/oil-type bases such as hydrophilic ointment and vanishing cream; and oil/water-type bases such as hydrophilic Vaseline, purified lanolin, Aquahole, Eucerin, Neocerin, hydrous lanolin, cold cream, and hydrophilic plastibase.

Any base may be used for the gel. Examples thereof include hydrogel base such as a carboxyvinyl polymer, a gel base, a fat-free ointment, polyvinylpyrrolidone, polyvinyl alcohol, sodium polyacrylate, carboxymethyl cellulose, starch, xanthane gum, karaya gum, sodium alginate, methylcellulose, hydroxypropyl cellulose, hydroxypropyl methylcellulose phthalate (HPMCP), cellulose acetate phthalate (CAP), carboxymethyl ethyl cellulose (CMEC), ethylcellulose, hydroxyethylcellulose, hydroxypropyl methylcellulose, carboxyvinyl polymer, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethyl cellulose, sodium carboxymethyl cellulose, calcium carboxymethyl cellulose, pullulan, chitosan, sodium carboxymethyl starch, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl aminoacetate, casein, alkyl alginate ester, gelatin, or polyethylene glycol.

Any base may be used for the poultice. Examples thereof include gelatin, sodium carboxymethyl cellulose, methylcellulose, sodium polyacrylate, kaolin, polyvinyl alcohol, polyvinylpyrrolidone, glycerin, propylene glycol, and water.

The tape preferably includes an adhesive layer containing formulation components (i.e., components such as the antigen, the first cellular immunity induction promoter, and if necessary, the second cellular immunity induction promoter), and a support that supports the adhesive layer. The tape may further include a release liner that prevents exposure of the adhesive layer before use and that can be easily removed from the adhesive layer at the time of use.

In the case where the vaccine pharmaceutical composition for transdermal administration of the present invention is in the form of a tape, the adhesive layer of the tape (hereinafter also referred to as the "tape of the present invention") contains an antigen, and, if desired, further contains the first cellular immunity induction promoter. The adhesive layer of the tape of the present invention contains an antigen in an amount of preferably 0.01 to 40% by weight, more preferably 0.1 to 30% by weight, based on the total weight of the adhesive layer. In the case where the adhesive layer of the tape of the present invention contains the first cellular immunity induction promoter, the amount of the cellular

immunity induction promoter is preferably 0.001 to 30% by weight, more preferably 0.01 to 20% by weight, based on the total weight of the adhesive layer.

Any adhesive may be used to form the adhesive layer. Examples of adhesives include acrylic adhesives formed from acrylic polymers; rubber-based adhesives containing a rubber elastomer such as a styrene-diene-styrene block copolymer (e. g. , styrene-isoprene-styrene block copolymer or styrene-butadiene-styrene block copolymer), polyisoprene, polyisobutylene, butyl rubber, or polybutadiene; silicone-based adhesives such as silicone rubber, dimethylsiloxane base, and diphenylsiloxane base; vinyl ether-based adhesives such as polyvinyl methyl ether, polyvinyl ethyl ether, and polyvinyl isobutyl ether; vinyl ester-based adhesives such as vinyl acetate-ethylene copolymer; and polyester-based adhesives formed from a carboxylic acid component such as dimethyl terephthalate, dimethyl isophthalate, or dimethyl phthalate, and a polyhydric alcohol component such as ethylene glycol. Particularly preferred adhesives are acrylic adhesive, rubber-based adhesives, and silicone-based adhesives. The amount (based on the solids content) of these adhesives in the adhesive layer is preferably 10 to 90% by weight, more preferably 20 to 80% by weight, based on the total weight of the adhesive layer.

Any acrylic adhesive may be used. Examples thereof include an acrylic acid ester-based adhesive containing, as a main component, a polymer containing a C2-C18 alkyl (meth)acrylate as a first monomer. Examples of the alkyl (meth) acrylate (first monomer) include an alkyl (meth) acrylate whose alkyl group is a C1-C18 linear, branched, or cyclic alkyl group (e.g., methyl, ethyl, propyl, butyl, pentyl, hexyl, cyclohexyl, heptyl, octyl, 2-ethylhexyl, nonyl, decyl, undecyl, dodecyl, or tridecyl). The alkyl (meth)acrylate (first monomer) is preferably an alkyl (meth)acrylate whose alkyl group is a C4-C18 linear, branched, or cyclic alkyl group (e.g. , butyl, pentyl, hexyl, cyclohexyl, heptyl, octyl, 2-ethylhexyl, nonyl, decyl, undecyl, dodecyl, or tridecyl). Further, since a monomer component that lowers the glass transition temperature of a polymer is suitably used to impart adhesiveness at room temperature, an alkyl (meth) acrylate whose alkyl group is a C4-C8 linear, branched, or cyclic alkyl group (e.g. , butyl, pentyl, hexyl, cyclohexyl, heptyl, octyl, or 2-ethylhexyl; preferably butyl, 2-ethylhexyl, or cyclohexyl; particularly preferably 2-ethylhexyl) is more preferred. Specifically, butyl acrylate, 2-ethylhexyl acrylate, 2-ethylhexyl methacrylate, cyclohexyl acrylate, cyclohexyl methacrylate, and the like are more preferred, and 2-ethylhexyl acrylate is the most preferred among these. These alkyl (meth)acrylates (first monomer components) may be used singly or in combination of two or more thereof.

The acrylic adhesive may also contain a second monomer capable of copolymerizing with the alkyl (meth)acrylate. Examples of the second monomer include a monomer having a functional group that can form a crosslinking point when a crosslinking agent is used. Examples of functional groups capable of being involved in crosslinking reaction include groups such as hydroxyl, carboxyl, and vinyl groups. Among these, hydroxyl and carboxyl groups are preferred. Specific examples of the monomer (second monomer component) include hydroxyethyl (meth)acrylate, hydroxypropyl (meth)acrylate, N-hydroxyalkyl (meth)acrylamide, (meth)acrylic acid, itaconic acid, maleic acid, maleic anhydride, mesaconic acid, citraconic acid, and glutaconic acid. Among these, acrylic acid, methacrylic acid, hydroxyethyl acrylate (particularly, 2-hydroxyethyl acrylate) are preferred, and acrylic acid is the most preferred, in view of easy availability. These monomers (second monomer components) may be used singly or in combination of two or more thereof.

In addition to the second monomer, a third monomer may be added to the acrylic adhesive, if desired. Example of the third monomer (third monomer component) include vinyl esters such as vinyl acetate and vinyl propionate; vinyl ethers such as methyl vinyl ether and ethyl vinyl ether; vinyl amides such as N-vinyl-2-pyrrolidone and N-vinylcaprolactam; alkoxy (meth)acrylates such as methoxyethyl (meth)acrylate, ethoxyethyl (meth)acrylate, and tetrahydrofurfuryl (meth)acrylate; hydroxyl group-containing monomers (these monomers are used as third monomer components and thus are not regarded as crosslinking points) such as hydorxypropyl (meth)acrylate and $\alpha$-hydroxymethyl acrylate; (meth)acrylic acid derivatives having an amide group such as (meth) acrylamide, dimethyl (meth)acrylamide, N-butyl (meth)acrylamide, and N-methylol (meth)acrylamide; aminoalkyl (meth)acrylates such as aminoethyl (meth)acrylate, dimethylaminoethyl (meth)acrylate, and t-butylaminoethyl (meth)acrylate; alkoxyalkylene glycol (meth)acrylates such as methoxyethylene glycol (meth)acrylate, methoxydiethylene glycol (meth)acrylate, methoxypolyethylene glycol (meth)acrylate, and methoxypolypropylene glycol (meth)acrylate; (meth) acrylonitrile; sulfonic acid-contining monomers such as styrene sulfonic acid, allyl sulfonic acid, sulfopropyl (meth)acrylate, (meth)acryloyloxy naphthalene sulfonic acid, acrylamide methyl sulfonic acid; and vinyl group-containing monomers such as vinylpiperidone, vinylpyrimidine, vinylpiperazine, vinylpyrrole, vinylimidazole, vinyloxazole, and vinylmorpholine. Preferred among these are vinyl esters and vinyl amides. Vinyl acetate is preferred among vinyl esters, and N-vinyl-2-pyrrolidone is preferred among vinyl amides. These monomers (third monomer components) may be used singly or in combination of two or more thereof.

In the case where the acrylic adhesive is a copolymer of an alkyl (meth)acrylate (first monomer component) and a vinyl monomer (second monomer component) having a functional group capable of being involved in crosslinking reaction, the alkyl (meth) acrylate and the vinyl monomer having a functional group capable of being involved in crosslinking reaction are preferably copolymerized at a weight ratio of 99-85:1-15, more preferably at a weight ratio of 99-90:1-10.

In addition, in the case where the acrylic adhesive is a copolymer of an alkyl (meth) acrylate (first monomer component), a vinyl monomer (second monomer component) having a functional group capable of being involved in crosslinking reaction, and a different monomer (third monomer component), the alkyl (meth)acrylate, the vinyl monomer

having a functional group capable of being involved in crosslinking reaction, and the different monomer are preferably copolymerized at a weight ratio of 40-94:1-15:5-50, more preferably at a weight ratio of 50-89:1-10:10-40.

The polymerization reaction may be carried out by any method known per se. For example, the above monomers may be reacted in the presence of an initiator (e.g. , benzoyl peroxide or azobisisobutyronitrile) in a solvent (e.g., ethyl acetate) at 50°C to 70°C for 5 to 48 hours.

Examples of particularly preferred acrylic adhesives in the present invention include 2-ethylhexyl acrylate/acrylic acid/N-vinyl-2-pyrrolidone copolymer, 2-ethylhexyl acrylate/N-(2-hydroxyethyl)acrylamide/N-vinyl-2-pyrrolidone copolymer, 2-ethylhexyl acrylate/2-hydroxyethyl acrylate/vinyl acetate copolymer, and 2-ethylhexyl acrylate/acrylic acid copolymer. 2-Ethylhexyl acrylate/acrylic acid/N-vinyl-2-pyrrolidone copolymer is more preferred.

If desired, these acrylic adhesives may be subjected to physical crosslinking treatment by radiation such as ultraviolet irradiation or electron beam irradiation, or chemical crosslinking treatment using various crosslinking agents such as an isocyanate-based compound such as trifunctional isocyanate, organic peroxide, organic metal salt, metal alcoholate, metal chelate compound, or polyfunctional compound (e.g., a polyfunctional external crosslinking agent or a polyfunctional monomer for internal crosslinking such as diacrylate or dimethacrylate).

The rubber-based adhesive is not particularly limited, and examples thereof include rubber adhesives containing a rubber elastomer such as polyisobutylene/polybutene-based elastomer, a styrene/diene/styrene block copolymer, styrene/butadiene-based elastomer, nitrile-based elastomer, chloroprene-based elastomer, vinylpyridine-based elastomer, polyisobutylene-based elastomer, butyl-based elastomer, or isoprene/isobutylene-based elastomer. Preferred among these are polyisobutylene (PIB) and a styrene/diene/styrene block copolymer (such as a styrene/butadiene/styrene block copolymer (SBS) or a styrene/isoprene/styrene block copolymer (SIS)), in view of solubility of the peptide and the cellular immunity induction promoter in the adhesive and the skin adhesiveness. These adhesives may be used in combination.

In addition, in order to achieve appropriate adhesion and drug solubility, the rubber-based adhesive may be a mixture of rubber elastomers formed from the same or different components and each having a different average molecular weight. For example, in the case of polyisobutylene, a mixture of high molecular weight polyisobutylene having an average molecular weight of 150,000 to 5,500,000 and medium molecular weight polyisobutylene having an average molecular weight of 10,000 to 150,000 and/or low molecular weight polyisobutylene having an average molecular weight of 500 to 4,000 is preferred. The amount of high molecular weight polyisobutylene is 10 to 80% by weight, preferably 20 to 70% by weight, relative to the total amount of polyisobutylene. The amount of medium molecular weight polyisobutylene is 0 to 90% by weight, preferably 10 to 80% by weight, relative to the total amount of polyisobutylene. The amount of low molecular weight polyisobutylene is 0 to 80% by weight, preferably 10 to 60% by weight, relative to the total amount of polyisobutylene.

As user herein, the term "average molecular weight" refers to a viscosity average molecular weight calculated from the Flory viscosity equation. The average molecular weight is determined by calculating the Staudinger index ($J_0$) from the flow time of the capillary 1 of an Ubbelohde viscometer at 20°C by the Schul-z-Blaschke equation, and using this $J_0$ value in the following expression.

$$J_0 = \eta_{sp}/c\ (1 + 0.31\eta_{sp})\ \text{(Schulz-Blaschke equation)}$$

$$\eta_{sp} = t/t_0 - 1$$

t: Flow time of solution (according to Hagenbach-couette correction formula)

$t_0$: Flow time of solvent (according to Hagenbach-couette correction formula)

c: Concentration of solution (g/cm$^3$)

$$J_0 = 3.06 \times 10^{-2}\overline{Mv}^{0.65}$$

$\overline{Mv}$ : Viscosity average molecular weight

In order to provide appropriate tackiness, the rubber-based adhesive may contain a tackifier such as rosin-based resin, polyterpene resin, chroman-indene resin, petroleum-based resin, terpene-phenol resin, xylene resin, or alicyclic saturated hydrocarbon resin. One or two or more of these tackifiers can be added to the rubber-based adhesive in an amount of 50% by weight or less, preferably 5 to 40% by weight, based on the total weight of the rubber-based adhesive.

Examples of the silicone-based adhesive include silicone-based adhesives mainly made of, for example, polyorganosiloxane, polydimethylsiloxane, or polydimethyldiphenyl-siloxane. In particular, commercially available silicone-based adhesives such as BIO PSA (Dow Corning Corporation) are preferred.

The support that supports the adhesive layer is not particularly limited, but preferably, it is one that is substantially impervious to the peptide and the cellular immunity induction promoter. In other words, it is preferably one that prevents a decrease in the amount of the peptide, the cellular immunity induction promoter, additives, or the like contained in the adhesive layer, which would otherwise occur if these components passed through the support and leaked from the rear surface.

The support may be a single film of polyester, polyamide, polyvinylidene chloride, polyethylene, polypropylene, polyvinyl chloride, ethylene-ethyl acrylate copolymer, polytetrafluoroethylene, ionomer resin, metal foil, or the like, or it may be a laminated film of these mentioned above. Preferred among these is a laminated film of a nonporous plastic film which are made of the above-mentioned materials and a porous film, in view of achieving good adhesiveness (anchoring properties) between the support and the adhesive layer. In this case, the adhesive layer is preferably formed on the porous film side. Porous films which improve the anchoring properties between the support and the adhesive layer are employed. Specific examples thereof include paper, woven fabrics, nonwoven fabrics, knitted fabrics, and mechanically perforated sheets. Particularly preferred among these are paper, woven fabrics, and nonwoven fabrics, in view of factors such as handleability. A porous film having a thickness in the range of 1 to 200 μm is employed in view of improving anchoring properties and also in view of factors such as flexibility and adhesion operability of the tape. In addition, in the case where the porous film is a woven fabric or a nonwoven fabric, the basis weight is preferably 5 to 30 $g/m^2$, more preferably 6 to 15 $g/m^2$.

The most suitable support is a laminated film of a polyester film (preferably, a polyethylene terephthalate film) having a thickness of 1.5 to 6 μm and a polyester (preferably, polyethylene terephthalate) nonwoven fabric having a basis weight of 6 to 15 $g/m^2$.

The tape of the present invention preferably includes a release liner laminated on an adhesive surface cf the adhesive layer in order to protect the adhesive surface until use. Any release liner may be used as long as it has been treated to be releasable and it can be released with a sufficiently small release force. Examples thereof include films made of polyester, polyvinyl chloride, polyvinylidene chloride, polyethylene terephthalate, or the like; paper such as fine paper and glassine paper; and laminate films of fine paper or glassine paper and polyolefin, which have been treated to be releasable by applying, for example, silicone resin or fluorine resin to the surface to be contacted with the adhesive layer. The thickness of the release liner is preferably 10 to 200 μm, more preferably 25 to 100 μm. The release liner is preferably formed from polyester (in particular, polyethylene terephthalate) resin in view of factors such as barrier and cost.

Also in this case, the thickness of the release liner is preferably about 25 to 100 μm in view of handleability.

In addition, the vaccine pharmaceutical composition of the present invention may contain an additive, if necessary. The additive is selected from, for example, isotonizing agents, antiseptics/germicides, antioxidants, resolvents, solubilizing agents, suspending agents, fillers, pH adjusting agents, stabilizers, absorption promoters, release rate controlling agents, colorant, plasticizer, crosslinking agent, and adhesive, or a combination of two or more thereof, according to factors such as the compatibility with the main component of the base, the antigen, and the cellular immunity induction promoter, and the intended administration regimen. In addition, in the case where the pharmaceutical composition of the present invention is in the form of a tape, the tape may contain a skin permeability enhancer as an additive.

As used herein, the term "skin permeability enhancer" refers to any substance that can improve permeation efficiency of a transdermally administered antigen through the skin, as compared to the efficiency obtained without the substance. Any skin permeability enhancer may be used as long as it is liquid, i.e., it is fluidic, at room temperature (25°C) and has an absorption promoting effect. When the skin permeability enhancer is a mixture of two or more substances, the resultant mixture is liquid at room temperature (25°C) and has an absorption promoting effect. The liquid component is preferably a hydrophobic liquid in view of compatibility with the adhesive layer.

Examples of the skin permeability enhancer include higher alcohols such as oleyl alcohol and octyl dodecanol; polyhydric alcohols such as glycerin, ethylene glycol, and polypropylene glycol; higher fatty acids such as oleic acid and caprylic acid; fatty acid esters such as isopropyl myristate, isopropyl palmitate, and ethyl oleate; polybasic acid esters such as diethyl sebacate and diisopropyl adipate; polyhydric alcohol fatty acid esters such as diglyceryl triisostearate, sorbitan monooleate, propylene glycol dicaprylate, polyethylene glycol monolaurate, and polyoxyethylene sorbit tetraoleate; polyoxyethylene alkyl ethers such as polyoxyethylene lauryl ether; hydrocarbons such as squalane and liquid paraffin; vegetable oils such as olive oil and castor oil; silicone oil; pyrrolidones such as N-methylpyrrolidone and N-dodecylpyrrolidone; and sulfoxides such as decylmethylsulfoxide. These may be used singly or in combination of two or more thereof.

In the case where rubber-based or acrylic adhesive is used, a second skin permeability enhancer may be used. Non-limiting specific examples of the second skin permeability enhancer include polyvinylpyrrolidone, crospovidone, polypropylene glycol, polyvinyl alcohol, carboxyvinyl polymers, hydroxypropyl cellulose, and mixtures thereof. In a preferred embodiment, the second skin permeability enhancer of the present invention is polyvinylpyrrolidone, crospovidone, and/or polypropylene glycol.

In view of enhancing skin permeation of the antigen peptide, examples of the skin permeability enhancer include higher alcohols (more specifically, C8-C18 (preferably C8-C14) higher alcohols), fatty acid esters (more specifically, fatty acid esters of C8-C18 (preferably C12-C16) fatty acids and C1-C18 monohydric alcohol), and polyhydric alcohol fatty acid esters. In particular, a fatty acid ester, specifically, isopropyl myristate, isopropyl palmitate, or diethyl sebacate is preferably used. The amount of the skin permeability enhancer based on the total weight of the adhesive layer is preferably 0.1% by weight to 70% by weight, more preferably 1% by weight to 65% by weight, still more preferably 5% by weight to 60% by weight. If the amount of the skin permeability enhancer is 0.1% by weight or more, a high transdermal absorption promoting effect can be achieved. If the amount is 70% by weight or less, it is advantageous in that high transdermal absorption properties can be achieved while a decrease in adhesion and cohesion of the adhesive layer as a whole is suppressed.

<Vaccine pharmaceutical composition for mucosal administration>

The vaccine pharmaceutical composition for mucosal administration of the present invention exerts a high cellular immunity inducing effect in mucosal administration of various antigens to the subject.

As used herein, the term "mucosal administration" as in the pharmaceutical composition for mucosal administration may refer to any formulation that is usually used for mucosal administration, for example, sublingual, transnasal, buccal, rectal, or vaginal administration. The composition may be, for example, a semisolid formulation such as a gel (jelly), a cream, an ointment, and a plaster; a solution; a solid formulation such as a powder, a fine granule, a granule, a film, a tablet, and an orally-disintegrating tablet; a mucosal spray formulation such as an aerosol; or an inhalant. Categories, definitions, properties, production processes, and the like of these formulations are well known in the relevant art. For example, see the Japanese Pharmacopoeia, 16th Edition. Any known material may be used for these formulations.

The amount of the antigen in the vaccine pharmaceutical composition for transmucosal administration of the present invention is not particularly limited, but the lower limit of the amount based on the total weight of the composition is preferably 0.00001% by weight, more preferably 0.0001% by weight, still more preferably 0.001% by weight, and the upper limit thereof is preferably 100% by weight, more preferably 10% by weight, still more preferably 1% by weight.

The amount of the first cellular immunity induction promoter in the vaccine pharmaceutical composition for transmucosal administration of the present invention is not particularly limited, but the lower limit of the amount per part by weight of the antigen is preferably 0.001 parts by weight, more preferably 0.01 parts by weight, still more preferably 0. 1 parts by weight, and the upper limit thereof is preferably 100000 parts by weight, more preferably 10000 parts by weight, still more preferably 1000 parts by weight.

The amount of the second cellular immunity induction promoter in the vaccine pharmaceutical composition for transmucosal administration of the present invention is not particularly limited, but the lower limit of the amount per part by weight of the antigen is preferably 0.001 parts by weight, more preferably 0.01 parts by weight, still more preferably 0. 1 parts by weight, and the upper limit thereof is preferably 100000 parts by weight, more preferably 10000 parts by weight, still more preferably 1000 parts by weight.

If the amount is less than the lower limit, the immunity inducing effect may be insufficient. If the amount is more than the upper limit, the safety may be compromised.

Any solvent may be used for the solution. Examples thereof include solvents such as an appropriate amount of water, ethanol, glycerin, or propylene glycol. The solution can be prepared by dispersing or dissolving the components in the solvent.

Any base may be used for the gel (jelly). Examples thereof include a carboxyvinyl polymer as a hydrogel base, a gel base, a fat-free ointment, polyvinylpyrrolidone, polyvinyl alcohol, sodium polyacrylate, carboxymethyl cellulose, starch, xanthane gum, karaya gum, sodium alginate, methylcellulose, hydroxypropyl cellulose, hydroxypropyl methylcellulose phthalate (HPMCP), cellulose acetate phthalate (CAP), carboxymethyl ethyl cellulose (CMEC), ethylcellulose, hydroxyethylcellulose, hydroxypropyl methylcellulose, a carboxyvinyl polymer, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethyl cellulose, sodium carboxymethyl cellulose, calcium carboxymethyl cellulose, pullulan, chitosan, sodium carboxymethyl starch, Plantago testa, galactomannan, eudragit, casein, alkyl alginate ester, gelatin, and polyethylene glycol. A fluidic gel and a formable gel can be prepared by dissolving these bases in a solvent. The solvent is preferably water, but glycerin or propylene glycol may be used.

Any base may be used for the cream. Examples thereof include water/oil-type bases such as hydrophilic ointment and vanishing cream; and oil/water-type bases such as hydrophilic Vaseline, purified lanolin, Aquahole, Eucerin, Neocerin, hydrous lanolin, cold cream, and hydrophilic plastibase. These bases are placed in an oil/fat solvent, and stirred at high speed using a homogenizer or the like, whereby a cream can be prepared.

Any base may be used for the film. Examples thereof include polyvinylpyrrolidone, polyvinyl alcohol, sodium polyacrylate, carboxymethyl cellulose, starch, xanthane gum, karaya gum, sodium alginate, methylcellulose, carboxyvinyl polymer, agar, hydroxypropyl cellulose, hydroxypropyl methylcellulose phthalate (HPMCP), cellulose acetate phthalate(CAP), carboxymethyl ethyl cellulose (CMEC), ethylcellulose, hydroxyethylcellulose, hydroxypropyl methylcellulose, carboxyvinyl polymer, tragacanth, gum arabic, locust bean gum, guar gum, carrageenan, dextrin, dextran, amylose, potassium carboxymethyl cellulose, sodium carboxymethyl cellulose, calcium carboxymethyl cellulose, pullulan, chitosan, sodium carboxymethyl starch, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl aminoacetate, casein, and alkyl alginate ester. These bases are dissolved in a polar organic solvent such as water or ethanol, applied to form a thin film, and dried, whereby a film can be prepared. In one preferred embodiment, the vaccine pharmaceutical composition for mucosal administration of the present invention is in the form of a film.

Additives for the powder, fine granule, granule, and tablets are not particularly limited. For example, these formulations can be prepared by mixing an excipient such as lactose, corn starch, or crystalline cellulose; a binder such as hydroxypropyl cellulose or gum arabic; and an appropriate amount of a solvent such as water or ethanol, and then subjecting the mixture to a combination of processes such as granulation, drying, and tableting. If necessary, a lubricant such as magnesium stearate or a coating agent such as hydroxypropyl cellulose or sucrose can also be added.

Any base may be used for the orally-disintegrating tablet (freeze dry type). Examples thereof include polysaccharides such as gelatin and pullulan. In addition, mannitol, trehalose, sorbitol, glycine, or the like may be used as a forming aid. The orally-disintegrating tablet (freeze dry type) can be prepared by dissolving the additive(s) in water to form a solution, dispensing the solution, and freeze drying the solution. In one preferred embodiment, the vaccine pharmaceutical composition for mucosal administration of the present invention is in the form of an orally-disintegrating tablet.

The aerosol is not particularly limited. The content of the aerosol may be, for example, a solution, a gel having high fluidity, cream, fine powder such as a powdered drug, or the like. The aerosol can be efficiently administered to a site of administration, for example, oral mucosa or nasal mucosa, by dispersing the solid or liquid fine particles in a gas with an atomizing device.

<Vaccine pharmaceutical composition for intradermal, subcutaneous, and intramuscular administration>

The vaccine pharmaceutical composition for intradermal, subcutaneous, or intramuscular administration of the present invention exerts a high cellular immunity inducing effect in intradermal, subcutaneous, and intramuscular administration of various antigens to the subject.

As used herein, the term "intradermal, subcutaneous, or intramuscular administration" as in the pharmaceutical composition for intradermal, subcutaneous, or intramuscular administration may refer to any formulation having fluidity to a degree that allows for injection, for example, a solution, a suspension, or a cream. Categories, definitions, properties, production processes, and the like of these formulations are well known in the relevant art. For example, see the Japanese Pharmacopoeia, 16th Edition. Any known material may be used for these formulations.

The amount of the antigen in the vaccine pharmaceutical composition for intradermal, subcutaneous, or intramuscular administration of the present invention is not particularly limited, but the lower limit thereof based on the total weight of the composition is preferably 0.0000001% by weight, more preferably 0.000001% by weight, still more preferably 0.00001% by weight, and the upper limit thereof is preferably 0.01% by weight, more preferably 0.001% by weight, still more preferably 0.0001% by weight.

The amount of the first cellular immunity induction promoter in the vaccine pharmaceutical composition for intradermal, subcutaneous, or intramuscular administration of the present invention is not particularly limited, but the lower limit thereof per part by weight of the antigen is preferably 1 part by weight, more preferably 10 parts by weight, still more preferably 200 parts by weight, and the upper limit thereof is preferably 1000000 parts by weight, more preferably 100000 parts by weight, still more preferably 10000 parts by weight.

The amount of the second cellular immunity induction promoter in the vaccine pharmaceutical composition for intradermal, subcutaneous, or intramuscular administration of the present invention is not particularly limited, but the amount thereof per part by weight of the antigen is preferably 1 part by weight, more preferably 10 parts by weight, still more preferably 200 parts by weight, and the upper limit thereof is preferably 10000000 parts by weight, more preferably 100000 parts by weight, still more preferably 10000 parts by weight.

If the amount is less than the lower limit, the immunity inducing effect may be insufficient. If the amount is more than the upper limit, the safety may be compromised.

Any solvent may be used for the solution. For example, an appropriate amount of water, saline, ethanol, glycerin, or propylene glycol can be used as a solvent, and a solution can be prepared by dispersing or dissolving components in any of these solvents.

Any base may be used for the aqueous suspension. Examples thereof include a hydrogel base such as a carboxyvinyl polymer, a gel base, a fat-free ointment, polyvinylpyrrolidone, polyvinyl alcohol, sodium polyacrylate, carboxymethyl cellulose, starch, xanthane gum, karaya gum, sodium alginate, methylcellulose, hydroxypropyl cellulose, hydroxypropyl methylcellulose phthalate (HPMCP), cellulose acetate phthalate (CAP), carboxymethyl ethyl cellulose (CMEC), ethylcellulose, hydroxyethylcellulose, hydroxypropyl methylcellulose, carboxyvinyl polymer, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethyl cellulose, sodium carboxymethyl cellulose, calcium carboxymethyl cellulose, pullulan, chitosan, sodium carboxymethyl starch, Plantago testa, galactomannan, eudragit, casein, alkyl alginate ester, gelatin, or polyethylene glycol. A fluidic suspension can be prepared by dissolving these bases in a solvent. The solvent is preferably saline, but glycerin or propylene glycol may be used.

Any base may be used for the hydrophobic suspension. Examples thereof include water/oil-type bases such as hydrophilic ointment and vanishing cream; and oil/water-type bases such as hydrophilic Vaseline, purified lanolin, Aquahole, Eucerin, Neocerin, hydrous lanolin, cold cream, and hydrophilic plastibase. These bases are placed in an oil/fat solvent, and stirred at high speed using a homogenizer or the like, whereby an oil/fat suspension can be prepared.

The vaccine pharmaceutical composition for intradermal, subcutaneous, or intramuscular administration may contain an additive, if necessary. The additive is selected from, for example, isotonizing agents, antiseptics/germicides, antioxidants, resolvents, solubilizing agents, suspending agents, fillers, pH adjusting agents, stabilizers, absorption promoters, release rate controlling agents, colorant, plasticizer, and adhesive, or a combination of two or more thereof, according to factors such as the compatibility with the main component of the base, the antigen, and the first cellular immunity induction promoter, and the intended administration regimen.

The therapeutically effective amount of the vaccine pharmaceutical composition of the present invention may widely vary, when administered to the subject, depending on severity of the disease, age and relative health of the subject and other known factors. Generally, satisfactory results can be obtained at a dose of about 0.1 μg to 1 g/kg body weight per day. The first cellular immunity induction promoter is simultaneously or sequentially administered with the antigen. Simultaneous administration is preferred. The effective amount of the first cellular immunity induction promoter may widely vary depending on the specific first cellular immunity induction promoter that is actually used and the presence or absence of other cellular immunity induction promoter (s) to be used. Generally, satisfactory results can be obtained at a dose of about 0.01 μg to 1 g/kg body weight per day.

The daily dose may be administered in one time, or may be split into multiple doses (i.e., two or more doses, for example, 2, 3, 4, or 5 doses). Preferably, the period of continuous administration per dose is appropriately selected in the range from 1 minute to 7 days. Preferably, the administration interval is appropriately selected from one time daily to yearly (for example, once a day, once two days, once three days, once a week, once two week, once a month, once three months, once six months, once a year, etc.), or longer administration intervals, depending on the condition of patients, severity of the disease, whether it is for therapeutic purposes or preventive purposes, or the like. Generally, for the therapeutic purposes for patients actually having a severe disease, the antigen is preferably administered in a higher frequency and/or with a higher dose, while for the preventive purposes for patients not having a disease, the antigen is preferably administered in a lower frequency and/or with a lower dose.

- Advantageous Effects of Invention

The vaccine pharmaceutical composition of the present invention induces antigen-specific CD8-positive T cells necessary for cellular immunity, and further suppresses induction of apoptosis of antigen-specific CD8-positive T cells, thus making it possible to effectively induce cellular immunity even in continuous multiple dose administration. In addition to subcutaneous injection, intradermal injection, and intramuscular injection, the vaccine pharmaceutical composition of the present invention can also be transdermally administered and transmucosally administered, which provides the following advantages: excellent compliance, for example, non-invasive administration, no pain, and release from fear of injection; patients can administer the vaccine pharmaceutical composition by themselves because the composition can be easily administered; a risk of accidental needlestick injury by health care workers can be avoided; in the case of repetitive administration, the ambulatory frequency can be reduced, and this can contribute to the improvement in quality of life of the patient; and medical wastes which necessitate special disposition such as an injection needle are not generated. In addition, in the case where the pharmaceutical composition of the present invention is in the form of a patch such as a poultice or a tape, it is advantageous in that a predetermined dose can be reliably administered; the drug release rate can be controlled at any rate; and the drug is prevented from being attached to a site other than the intended site. Further, since a patch is easily detachable, it is advantageous in that patients can immediately discontinue administration on their own by removing the patch from the site of application when an adverse effect occurs, for example. Still another advantage is that the effects of the vaccine pharmaceutical composition of the present invention are significantly improved, as compared to the case where the antigen is administered by itself.

The vaccine pharmaceutical composition of the present invention is effective for cancer care including treatment and prevention.

BRIEF DESCRIPTION OF DRAWINGS

Fig. 1 shows diagrams showing expression of PD-1 receptor in antigen-specific CD8-positive T cells resulting from subcutaneous administration of injections prepared in Example 3 and Comparative Example 6.

DESCRIPTION OF EMBODIMENTS

The present invention will be described below in further detail with reference to examples, but the present invention is not limited to these examples.

(Examples 1, 2)

(Preparation of cream for transdermal administration)

A cream for transdermal administration was prepared according to the formulation shown in Table 2 below. Specifically, an antigen peptide, a first cellular immunity induction promoter, a second cellular immunity induction promoter (helper peptide), 15% by weight of dimethylsulfoxide (DMSO), which are described below, were mixed in amounts as specified in Table 2. A base (base cream) was added to the mixture to obtain a total weight of 100% by weight, followed by mixing. Thus, a cream for transdermal administration was prepared. The base cream used was prepared by mixing the materials shown in Table 1 in amounts as specified.

A PET film/PET nonwoven fabric laminate (area: 0.7 $cm^2$) was attached to the center of an adhesive tape for fixing, with the PET film side on the tape side, whereby a complex base was provided. The cream for transdermal administration (4 mg) was applied to a nonwoven fabric portion of the complex base. This was used as an administration sample in an immunity test.

[Table 1]

| Additive | Amount |
|---|---|
| White Vaseline | 60.7% by weight |
| Sorbitan monostearate | 0.7% by weight |
| Isostearic acid | 12.0% by weight |
| Benzyl alcohol | 2.4% by weight |
| Cetanol | 2.4% by weight |
| Stearyl alcohol | 3.5% by weight |
| Polysorbate 60 | 3.5% by weight |
| Concentrated glycerin | 2.4% by weight |
| Purified water | 12.4% by weight |

White Vaseline, sorbitan monostearate, isostearic acid, benzyl alcohol, stearyl alcohol, Polysorbate 60, concentrated glycerin, and dimethylsulfoxide (DMSO) were purchased from Wako Pure Chemical Industries, Ltd. Cetanol was purchased from Tokyo Chemical Industry Co., Ltd. Imiquimod (IMQ) as the first cellular immunity induction promoter was purchased from Ferrer, and loxoprofen sodium (LOX) was purchased from Yoshindo Inc. PADRE was used as the second cellular immunity induction promoter.

Note that OVA peptide is an amino acid peptide having a sequence of SIINFEKL (Ser Ile Ile Asn Phe Glu Lys Leu) (SEQ ID No: 16).

<Evaluation 1>

The creams for transdermal administration prepared in the examples were evaluated as follows.

Mouse immunity test 1 (cream)

According to the procedure described below, the cream for transdermal administration was used to carry out a mouse immunity test using an animal model for immunological evaluation. Subsequently, the level of induction of

antigen-specific cellular immunity was evaluated by ELISPOT assay.

Specifically, the back of a mouse was shaved, and the mouse was reared for a while to recover from skin damage caused by shaving. Then, the sample was administered to the skin on the back of the mouse for a certain period of time, and removed. The mouse was reared for a certain period of days, and the level of induction of antigen-specific cellular immunity was evaluated. The spleen was extracted a predetermined days after the last administration, and a spleen cell suspension was prepared. Spleen cells ($1 \times 10^6$ cells/well) and the antigen peptide (100 $\mu$M) in a culture medium were poured into wells of an ELISPOT plate on which anti-mouse IFN-$\gamma$-antibody was immobilized. The spleen cells were co-cultured with the antigen peptide for 20 hours under culture conditions of 37°C and 5% $CO_2$. The number of IFN-$\gamma$-producing cell spots (number of spots/$1 \times 10^6$ cells) was evaluated by ELISPOT assay. The cream was administered in a dose of 4 mg every time at a frequency of (24 hr/week) $\times$ one time or (24 hr/week) $\times$ two times. The spleen was extracted 6 days after the last administration. The "(24 hr/week)" means that transdermal administration was carried out continuously for 24 hours per week.

Further, the number of IFN-$\gamma$-producing cell spots from the second immunization relative to the number of IFN-$\gamma$-producing cell spots from the first immunization was measured as a ratio of increase or decrease in IFN-$\gamma$-producing cells. Table 2 shows the results.

(Examples 3 to 5, Comparative Examples 1 to 6)

(Preparation of injection for subcutaneous administration)

An injection for subcutaneous administration was prepared according to the formulation shown in Table 2 below. This was used as an administration sample in an immunity test. Specifically, the antigen, the first cellular immunity induction promoter, and the second cellular immunity induction promoter (helper peptide) were weighed and mixed in amounts as specified in Table 2, and saline was added to the mixture to obtain a total weight of 1000 $\mu$L, followed by mixing with a homogenizer. Thus, an injection for subcutaneous administration was prepared.

<Evaluation 2>

The injections for subcutaneous administration prepared in the examples were evaluated as follows.

Mouse immunity test 2 (injection)

According to the following procedure, the injection for subcutaneous administration was used to carry out a mouse immunity test using an animal model for immunological evaluation.

Specifically, a mouse immunity test was carried out as in the mouse immunity test 1 described above. The injection was subcutaneously administered in a dose of 50 $\mu$L at a frequency of one or two times. The spleen was extracted 6 days after the last administration. Table 2 shows the results.

Mouse skin permeability test (cream)

OVA peptide, imiquimod (IMQ), and loxoprofen (LOX) were tested for skin permeability using a Franz type diffusion cell. The skin isolated from the back of a mouse that had been shaved in advance was mounted on the Franz type diffusion cell (application area: 4.91 $cm^2$) in which phosphate buffer (pH 7.4 isotonic buffer) at 37°C was circulated. A preparation having a size of 0.7 $cm^2$ of was attached to the skin mounted on the Franz type diffusion cell, and a sample in the cell was collected 24 hours after the administration. The collected sample was subjected to a high performance liquid chromatograph-tandem mass spectrometer, and the amount of OVA peptide which permeated the skin 24 hours after the administration (amount of permeated OVA peptide, $\mu g/cm^2/24$ hr) and the amount of LOX (amount of permeated LOX, $\mu g/cm^2/24$ hr) were calculated from a calibration curve which had been determined in advance. Table 2 shows the results.

Weight ratio of the first cellular immunity induction promoter to the antigen

The weight ratio of the first cellular immunity induction promoter to the antigen in the injection administered was calculated from the dose injected into the immunized mouse. In the case of transdermal administration, none of the transdermally administered antigen and the transdermally administered first cellular immunity induction promoter are present in the skin. Since the preparation is removed 24 hours after the transdermal administration, the amounts of the antigen and the first cellular immunity induction promoter which permeated the skin were measured 24 hours after the mouse skin permeability test described above, and the weight ratio of the administered first cellular immunity induction

promoter to the administered antigen was calculated. Table 2 shows the results.

Method for testing the expression rate of PD-1 receptor in antigen-specific CD8-positive T cells

The expression rate of PD-1 receptor in antigen-specific CD8-positive T cells was evaluated by FACS (Becton, Dickinson and Company, Japan). A mouse immunity test was carried out in the same manner as in the mouse immunity test 1. The cream was administered in a dose of 4 mg, and the injection was administered in a dose of 50 μL, at a frequency of (24 hr/week) × two times. The spleen was extracted 3 days after the last administration. The "(24 hr/week)" means that transdermal administration was carried out continuously for 24 hours per week.

Further, the spleen cells were reacted with anti-CD8-FITC and tetramer-SIINFEKL-PE to detect OVA peptide-specific CD8-positive T cells, and reacted with anti-CD279 (PD-1 receptor)-APC to detect PD-1 receptor, and the expression rate was measured by FACS. Evaluation was carried out as follows: first, CD8-positive T cells and tetramer-positive (both positive) cells were gated; the percentage of PD-1 receptor-positive cells in these cells was measured, and the expression rate of PD-1 receptor in OVA peptide-specific CD8-positive T cells was evaluated. Fig. 1 shows representative results, and Table 2 shows all the results. Anti-CD8-FITC and tetramer-SIINFEKL-PE were purchased from MBL, and anti-CD279 (PD-1 receptor)-APC was purchased from Biolegend.

In the case of evaluating the expression rate of PD-1 receptor in antigen-specific CD8-positive T cells for antigens other than OVA peptide, the expression rate is measured using antigen-specific tetramer which is prepared or purchased instead of tetramer-SIINFEKL-PE.

In Examples 1 to 5, CTL was enhanced by two-time administration, and the expression rate of PD-1 receptor in antigen-specific CD8-positive T cells was low. In contrast, the opposite behavior was observed in Comparative Examples 1 to 6. This shows that the pharmaceutical composition can suppress expression of PD-1 receptor, and can enhance antigen-specific CD8-positive T cells in continuous multiple dose administration. In addition, the expression level of PD-1 receptor was low when the weight ratio of the administered first cellular immunity induction promoter was 150 parts by weight or more per part by weight the administered antigen. Table 2 below shows the test results.

Method for testing the rate of induction of apoptosis of antigen-specific CD8-positive T cells

The rate of induction of apoptosis of antigen-specific CD8-positive T cells was evaluated by FACS. A mouse immunity test was carried out in the same manner as in the mouse immunity test 1. The cream was administered in a dose of 4 mg, and the injection was administered in a dose of 50 μL, at a frequency of (24 hr/week) × two times. The spleen was extracted 3 days after the last administration. The "(24 hr/week)" means that transdermal administration was carried out continuously for 24 hours per week.

Further, the spleen cells were reacted with anti-CD8-FITC and tetramer-SIINFEKL-PE to detect OVA peptide-specific CD8-positive T cells, and reacted with APC-Annexin V to detect apoptosis, and the expression rate was measured by FACS. Evaluation was carried out as follows: first, CD8 positive T cells and tetramer positive (both positive) cells were gated; the percentage of Annexin V positive cells in these cells was measured; and the rate of induction of apoptosis of OVA peptide-specific CD8-positive T cells was evaluated. Fig. 1 shows representative results, and Table 2 shows all the results. Anti-CD8-FITC and tetramer-SIINFEKL-PE were purchased from MBL, and APC-Annexin V was purchased from Biolegend.

In the case of evaluating the rate of induction of apoptosis of antigen-specific CD8-positive T cells for antigens other than OVA peptide, the expression rate is measured using antigen-specific tetramer that is prepared or purchased instead of tetramer-SIINFEKL-PE.

In Examples 1 to 5, CTL was enhanced by two-time administration, and the rate of induction of apoptosis of antigen-specific CD8-positive T cells was low. In contrast, the opposite behavior was observed in Comparative Examples 1 to 6. This shows that the vaccine pharmaceutical composition of the present invention can suppress induction of apoptosis of CTL, and can enhance antigen-specific CD8-positive T cells in continuous multiple dose administration. In addition, the induction of apoptosis was low when the weight ratio of the administered first cellular immunity induction promoter was 150 parts by weight or more per part by weight of the administered antigen. Table 2 below show the test results.

Creams for transdermal administration, injections for intradermal administration, and injections for subcutaneous administration were prepared according to the formulations shown in Tables 3, 4, 5, and 6 below, in the same manner as in Examples 1 to 5 and Comparative Examples 1 to 6. In Table 3 below, the helper peptide was OVA class II peptide (sequence Ile Ser Gln Ala Val His Ala Ala His Ala Glu Ile Asn Glu Ala Gly Arg (SEQ ID No: 17)). In Table 4 below, the cancer antigen peptide was PR1 peptide. In Table 5 below, the cancer antigen peptide was HER2/neu_E75 peptide. In Table 6 below, the cancer antigen peptide was survivin 2B peptide.

In Examples 6 to 16 in Tables 3 to 6 below, according to the results of the method for testing the expression rate of PD-1 receptor in antigen-specific CD8-positive T cells, CTL was enhanced by two-time administration, and the

expression rate of PD-1 receptor in antigen-specific CD8-positive T cells was decreased. In contrast, the opposite behavior was observed in Comparative Examples 7 to 21. Thus, the pharmaceutical composition can suppress expression of PD-1 receptor, and can enhance antigen-specific CD8-positive T cells in continuous multiple dose administration. In addition, the expression level of PD-1 receptor decreases when the weight ratio of the administered first cellular immunity induction promoter was 150 parts by weight or more per part by weight of the administered antigen.

In addition, in Examples 6 to 16 in Tables 3 to 6 below, according to the results of the method for testing the rate of induction of apoptosis of antigen-specific CD8-positive T cells, CTL was enhanced by two-time administration in the examples, and the rate of induction of apoptosis of antigen-specific CD8-positive T cells was decreased. In contrast, the opposite behavior was observed in Comparative Examples 7 to 21. Thus, the pharmaceutical composition can suppress induction of apoptosis of CTL, and can enhance antigen-specific CD8-positive T cells in continuous multiple dose administration. In addition, the induction of apoptosis decreases when the weight ratio of the administered first cellular immunity induction promoter to the administered antigen was 150 parts by weight or more.

(Method for testing the antigen-specific Th1/Treg ratio)

The antigen-specific Th1/Treg ratio can be measured according to the procedure described below. For example, in the case of OVA antigen, a cream for transdermal administration and an injection for intradermal administration are prepared according to the formulation shown in Table 3 below, and evaluation can be made by carrying out an immunity test.

The Th1/Treg ratio is evaluated by FACS. A mouse immunity test is carried out in the same manner as in the mouse immunity test 1. The cream is administered in a dose of 4 mg, and the injection is intradermally administered in a dose of 50 $\mu$L, at a frequency of (1 to 3 times/week) $\times$ multiple times (2 to 5 times). The spleen is extracted 6 days or an appropriate number of days after the last administration.

The method for measuring the antigen-specific Th1/Treg ratio is as follows: in the case of Th1 cells, spleen cells are reacted with anti-CD4-FITC, OVA class II peptide-specific tetramer (or tetramer-ISQAVHAAHAEINEAGR-PE in the case of OVA antigen) and APC-CD183, and cells that are CD4 positive and tetramer positive (both positive) are gated by FACS. Then, the percentage of CD183 positive cells in these cells is measured, and the number of OVA class II peptide-specific Th1 cells is calculated. Treg cells are isolated from spleen cells using Dynabeads (DYNAL). Further, Treg cells are labeled with OVA class II peptide-specific tetramer (or tetramer-ISQAVHAAHAEINEAGR-PE in the case of OVA antigen), and the number of OVA class II peptide-specific Treg cells is calculated. Alternatively, Treg cells are labeled with anti-CD4-FITC, anti-FoxP3-APC, and tetramer-ISQAVHAAHAEINEAGR-PE), and the number of OVA class II peptide-specific Treg cells is calculated. The ratio of Th1 cells to Treg cells is calculated based on these cell counts. Anti-CD4-FITC and tetramer-ISQAVHAAHAEINEAGR-PE are purchased from MBL; aPC-CD183, anti-CD4-FITC, and anti-FoxP3-APC are purchased from Biolegend; and Dynabeads mouse $CD4^+CD25^+$Treg cells are purchased from Invitrogen. In the case of an antigen other than OVA, antigen-specific tetramer which is prepared or purchased is used.

[Table 2]

| | Route | Base | Antigen peptide | | | Second cellular immunity induction promoter ( helper peptide ) | | First cellular immunity induction promoter (other than helper peptide ) | | | Results of skin permeability test | | | Weight ratio of promoter to angien [%] { Cream: (D) or (E)/(C) } { Injection: (B)/(A) } | Number of IFN-γ-producing cell spots | | Increase/decrease rate of CTL [%] due to multiple dose administration { (G)/(F)*100 } | Percentage of expression level of PD-1 in CD8-positive and tetramer-positive cells [%] | Percentage of Annexin V+ in $CD8^+$ and tetramer+ cells [%] |
|---|---|---|---|---|---|---|---|---|---|---|---|---|---|---|---|---|---|---|---|
| | | | Kind | % by weight | Dose (ug) (A) | Kind | % by weight | Kind | % by weight | Dose (ug) (B) | OVA peptide Skin permeation amount (C) [ug/cm²/24h] | IMQ Skin permeation amount (D) [ug/cm²/24h] | LOX Skin permeation amount (E) [ug/cm²/24h] | | After 1st immunization (F) | After 2nd immunization (G) | | | |
| Example 1 | Transdermal | Cream | OVA peptide | 5 | 200 | PADRE | 1 | IMQ | 3 | 120 | 0.012 | 2 | - | 167 | 422 | 431 | 102.1 | 20 | 26 |
| Example 2 | | Cream | OVA peptide | 5 | 200 | PADRE | 1 | LOX | 3 | 120 | 0.015 | - | 40 | 2667 | 321 | 340 | 106.1 | 22 | 22 |
| Example 3 | Injection (sub-cutaneous) | Saline/Montanide | OVA peptide | 0.00008 | 0.04 | PADRE | 0.05 | IMQ | 0.05 | 25 | - | - | - | 625 | 632 | 745 | 118 | 24 | 14 |
| Comparative Example 1 | | Saline/Montanide | OVA peptide | 0.0004 | 0.2 | PADRE | 0.05 | IMQ | 0.05 | 25 | - | - | - | 125 | 834 | 559 | 67 | 48 | 31 |
| Comparative Example 2 | | Saline/Montanide | OVA peptide | 0.002 | 1 | PADRE | 0.05 | IMQ | 0.05 | 25 | - | - | - | 25 | 1032 | 462 | 45 | 42 | 32 |
| Comparative Example 3 | | Saline/Montanide | OVA peptide | 0.2 | 100 | PADRE | 0.05 | IMQ | 0.05 | 25 | - | - | - | 0.3 | 971 | 561 | 58 | 70 | 40 |
| Example 4 | | Saline/Montanide | OVA peptide | 0.000016 | 0.008 | PADRE | 0.05 | LOX | 0.05 | 25 | - | - | - | 3125 | 24 | 109 | 447 | 25 | 22 |
| Example 5 | | Saline/Montanide | OVA peptide | 0.00008 | 0.04 | PADRE | 0.05 | LOX | 0.05 | 25 | - | - | - | 625 | 64 | 135 | 211 | 29 | 28 |
| Comparative Example 4 | | Saline/Montanide | OVA peptide | 0.0004 | 0.2 | PADRE | 0.05 | LOX | 0.05 | 25 | - | - | - | 125 | 303 | 149 | 49 | 44 | 38 |
| Comparative Example 5 | | Saline/Montanide | OVA peptide | 0.002 | 1 | PADRE | 0.05 | LOX | 0.05 | 25 | - | - | - | 25 | 446 | 100 | 22 | 42 | 38 |
| Comparative Example 6 | | Saline/Montanide | OVA peptide | 0.2 | 100 | PADRE | 0.05 | LOX | 0.05 | 25 | - | - | - | 0.3 | 291 | 183 | 56 | 52 | 45 |

[Table 3]

| | Route | Base | Antigen peptide | | | Second cellular immunity induction promoter (helper peptide) | | First cellular immunity induction promoter (other than helper peptide) | | | Results of skin permeability test | | | Weight ratio of promoter to angiten [%] {Cream: (D) or (E)/(C)} {Injection: (B)/(A)} |
|---|---|---|---|---|---|---|---|---|---|---|---|---|---|---|
| | | | Kind | % by weight | Dose (ug) (A) | Kind | % by weight | Kind | % by weight | Dose (ug) (B) | OVA peptide Skin permeation amount (C) [ug/cm$^2$/24h] | IMQ Skin permeation amount (D) [ug/cm$^2$/24h] | LOX Skin permeation amount E [ug/cm$^2$/24h] | |
| Example 6 | Transdermal | Cream | OVA peptide | 5 | 200 | OVA class II peptide | 1 | IMQ | 3 | 120 | 0.012 | 2 | - | 167 |
| Example 7 | | Cream | OVA peptide | 5 | 200 | OVA class II peptide | 1 | LOX | 3 | 120 | 0.015 | - | 42 | 2800 |
| Example 8 | Injection (subcutaneous) | Saline/Montanide | OVA peptide | 0.00008 | 0.04 | OVA class II peptide | 0.1 | IMQ | 0.05 | 25 | - | - | - | 625 |
| Comparative Example 7 | | Saline/Montanide | OVA peptide | 0.0004 | 0.2 | OVA class II peptide | 0.1 | IMQ | 0.05 | 25 | - | - | - | 125 |
| Comparative Example 8 | | Saline/Montanide | OVA peptide | 0.002 | 1 | OVA class II peptide | 0.1 | IMQ | 0.05 | 25 | - | - | - | 25 |
| Comparative Example 9 | | Saline/Montanide | OVA peptide | 02 | 100 | OVA class II peptide | 0.1 | IMQ | 0.05 | 25 | - | - | - | 0.3 |
| Example 9 | | Saline/Montanide | OVA peptide | 0.000016 | 0.008 | OVA class II peptide | 0.1 | LOX | 0.05 | 25 | - | - | - | 3125 |
| Example 10 | | Saline/Montanide | OVA peptide | 0.00008 | 0.04 | OVA class II peptide | 0.1 | LOX | 0.05 | 25 | - | - | - | 625 |

(continued)

| | Route | Base | Antigen peptide | | | Second cellular immunity induction promoter (helper peptide) | | First cellular immunity induction promoter (other than helper peptide) | | | Results of skin permeability test | | | Weight ratio of promoter to angiten [%] {Cream: (D) or (E)/(C)} {Injection: (B)/(A)} |
|---|---|---|---|---|---|---|---|---|---|---|---|---|---|---|
| | | | Kind | % by weight | Dose (ug) (A) | Kind | % by weight | Kind | % by weight | Dose (ug) (B) | OVA peptide Skin permeation amount (C) [ug/cm$^2$/24h] | IMQ Skin permeation amount (D) [ug/cm$^2$/24h] | LOX Skin permeation amount E [ug/cm$^2$/24h] | |
| Comparative Example 10 | | Saline/Montanide | OVA peptide | 0.0004 | 02 | OVA class II peptide | 0.1 | LOX | 0.05 | 25 | - | - | - | 125 |
| Comparative Example 11 | | Saline/Montanide | OVA peptide | 0.002 | 1 | OVA class II peptide | 0.1 | LOX | 0.05 | 25 | - | - | - | 25 |
| Comparative Example 12 | | Saline/Montanide | OVA peptide | 0.2 | 100 | OVA class II peptide | 0.1 | LOX | 0.05 | 25 | - | - | - | 0.3 |

[Table 4]

| | Route | Base | Antigen peptide | | | Second cellular immunity induction promoter (helper peptide) | | First cellular immunity induction promoter (other than helper peptide) | | |
|---|---|---|---|---|---|---|---|---|---|---|
| | | | Kind | % by weight | Dose (ug) (A) | Kind | % by weight | Kind | % by weight | Dose (ug) (B) |
| Example 11 | Transdermal | Cream | PR1 peptide | 5 | 200 | PADRE | 1 | IMQ | 3 | 120 |
| Example 12 | Injection (subcutaneous) | Saline/Montanide | PR1 peptide | 0.00008 | 0.04 | PADRE | 0.05 | IMQ | 0.05 | 25 |
| Comparative Example 13 | | Saline/Montanide | PR1 peptide | 0.0004 | 0.2 | PADRE | 0.05 | IMQ | 0.05 | 25 |
| Comparative Example 14 | | Saline/Montanide | PR1 peptide | 0.002 | 1 | PADRE | 0.05 | IMQ | 0.05 | 25 |
| Comparative Example 15 | | Saline/Montanide | PR1 peptide | 0.2 | 100 | PADRE | 0.05 | IMQ | 0.05 | 25 |

[Table 5]

| | Route | Base | Antigen peptide | | | Second cellular immunity induction promoter (helper peptide) | | First cellular immunity induction promoter (other than helper peptide) | | |
|---|---|---|---|---|---|---|---|---|---|---|
| | | | Kind | % by weight | Dose (ug) (A) | Kind | % by weight | Kind | % by weight | Dose (ug) (B) |
| Example 13 | Transdermal | Cream | HER2/neu_E75 peptide | 5 | 200 | PADRE | 1 | IMQ | 3 | 120 |
| Example 14 | Injection (subcutaneous) | Saline/Montanide | HER2/neu_E75 peptide | 0.00008 | 0.04 | PADRE | 0.05 | IMQ | 0.05 | 25 |
| Comparative Example 16 | | Saline/Montanide | HER2/neu_E75 peptide | 0.0004 | 0.2 | PADRE | 0.05 | IMQ | 0.05 | 25 |
| Comparative Example 17 | | Saline/Montanide | HER2/neu_E75 peptide | 0.002 | 1 | PADRE | 0.05 | IMQ | 0.05 | 25 |
| Comparative Example 18 | | Saline/Montanide | HER2/neu_E75 peptide | 0.2 | 100 | PADRE | 0.05 | IMQ | 0.05 | 25 |

[Table 6]

| | Route | Base | Antigen peptide | | | Second cellular immunity induction promoter (helper peptide) | | First cellular immunity induction promoter (other than helper peptide) | | |
|---|---|---|---|---|---|---|---|---|---|---|
| | | | Kind | % by weight | Dose (ug) (A) | Kind | % by weight | Kind | % by weight | Dose (ug) (B) |
| Example 15 | Transdermal | Cream | Survivin 2B peptide | 5 | 200 | PADRE | 1 | IMQ | 3 | 120 |
| Example 16 | Injection (subcutaneous) | Saline/Montanide | Survivin 2B peptide | 0.00008 | 0.04 | PADRE | 0.05 | IMQ | 0.05 | 25 |
| Comparative Example 19 | | Saline/Montanide | Survivin 2B peptide | 0.0004 | 0.2 | PADRE | 0.05 | IMQ | 0.05 | 25 |
| Comparative Example 20 | | Saline/Montanide | Survivin 2B peptide | 0.002 | 1 | PADRE | 0.05 | IMQ | 0.05 | 25 |
| Comparative Example 21 | | Saline/Montanide | Survivin 2B peptide | 0.2 | 100 | PADRE | 0.05 | IMQ | 0.05 | 25 |

INDUSTRIAL APPLICABILITY

The vaccine pharmaceutical composition of the present invention can be universally used for induction of cellular immunity to various antigens and exerts a high cellular immunity inducing effect. The vaccine pharmaceutical composition effectively exerts a cellular immunity inducing effect even in continuous multiple dose administration.

## Claims

1. A vaccine pharmaceutical composition for administration containing an antigen to induce cellular immunity, wherein the vaccine pharmaceutical composition suppresses apoptosis of CTL or inhibits suppression of induction of CTL.

2. The vaccine pharmaceutical composition according to claim 1, wherein the expression rate of PD-1 receptor in antigen-specific CD8-positive T cells in an animal model for immunological evaluation administered with the vaccine pharmaceutical composition is 40% or less.

3. The vaccine pharmaceutical composition according to claim 1 or 2, wherein the rate of induction of apoptosis of antigen-specific CD8-positive T cells in an animal model for immunological evaluation administered with the vaccine pharmaceutical composition is 30% or less.

4. The vaccine pharmaceutical composition according to claim 1, wherein an antigen-specific Th1/Treg ratio in an animal model for immunological evaluation administered with the vaccine pharmaceutical composition is controlled.

**5.** The vaccine pharmaceutical composition according to claim 1, 2, 3, or 4, further containing a first cellular immunity induction promoter.

**6.** The vaccine pharmaceutical composition according to claim 1, 2, 3, 4, or 5, which is administered in multiple doses.

**7.** The vaccine pharmaceutical composition according to claim 5 or 6,
wherein the first cellular immunity induction promoter is at least one of a TLR ligand or a cyclooxygenase inhibitor.

**8.** The vaccine pharmaceutical composition according to claim 1, 2, 3, 4, 5, 6, or 7, further containing a second cellular immunity induction promoter which is a helper peptide.

FIG.1

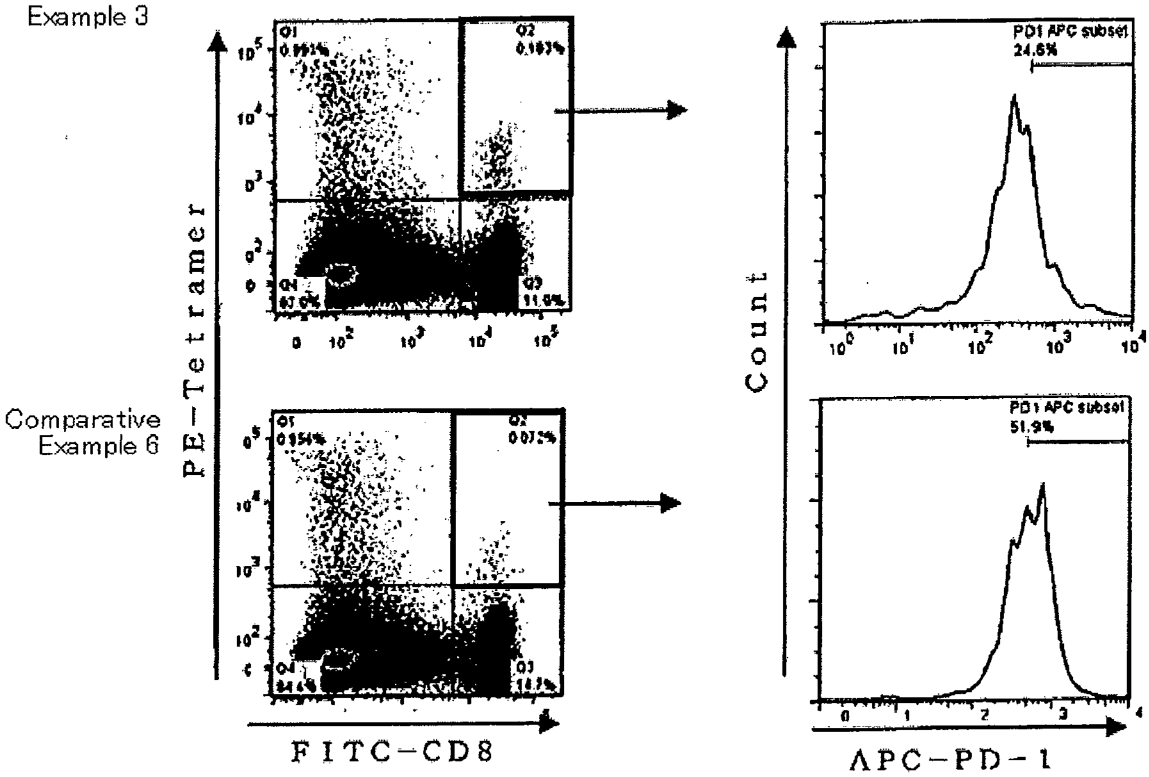
Example 3
Comparative Example 6
PE-Tetramer
FITC-CD8
Count
APC-PD-1
PD1 APC subset
24.6%
51.9%

## INTERNATIONAL SEARCH REPORT

International application No.
PCT/JP2015/072109

A. CLASSIFICATION OF SUBJECT MATTER
*A61K39/00*(2006.01)i, *A61K39/39*(2006.01)i, *A61K45/00*(2006.01)i, *A61P31/00*(2006.01)i, *A61P31/12*(2006.01)i, *A61P35/00*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K39/00, A61K39/39, A61K45/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | DAFTARIAN,P., et al., "Eradication of established HPV 16-expressing tumors by a single administration of a vaccine composed of a liposome-encapsulated CTL-T helper fusion peptide in a water-in-oil emulsion", Vaccine, 2006.06.12, Vol.24, No.24, p.5235-5244, ISSN 0264-410X, Abstract, Fig. 5, 7, 8, page 5241, right column to page 5243, left column, '4. Discussion' | 1-5,7,8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 October 2015 (29.10.15) | 10 November 2015 (10.11.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No. PCT/JP2015/072109

C (Continuation). DOCUMENTS CONSIDERED TO BE RELEVANT

| Category* | Citation of document, with indication, where appropriate, of the relevant passages | Relevant to claim No. |
|---|---|---|
| X | MURAOKA,D., et al., "Peptide Vaccine Induces Enhanced Tumor Growth Associated with Apoptosis Induction in CD8+ T Cells", J. Immunol., 2010.09.15, Vol.185, No.6, p.3768-3776, ISSN 1550-6606, Abstract, page 3769, left column, 'Immunization', page 3774, 'Combination of peptide vaccination with an adjuvant, a TLR9 ligand CpG, abrogates the elevated expression of PD-1 and Fas and induces strong antitumor immunity', FIGURE 4A,4B | 1-3,5-7 |
| X | Hiroshi SHIKU et al., "Peptide Vaccine ni yoru Men'eki Kiko no Kasseika", Mebio, 09 November 2010 (09.11.2010), vol.27, no.12, pages 28 to 35, ISSN 0910-0474, page 34, 'Gan Peptide Vaccine no Yukosei no Kaizen: Adjuvant no Heiyo', fig. 5 | 1-3,5-7 |
| X | Tsuyoshi ITO et al., "O-31 Toll like receptor-7 ligand o Riyo shita Keihiteki Kogen Kansa ni yoru Gan Vaccine Ryoho", Biotherapy, 31 October 2005 (31.10.2005), vol.19, suppl.1, page 114, ISSN 0914-2223, entire text | 1-3,5-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H07505883 T
- JP 2007529531 T
- US 20080286296 A
- US 20080193487 A


### Non-patent literature cited in the description


- **DAISUKE MURAOKA et al.** *The Journal of Immunology,* 2010, vol. 185, 3768-3776
- **YOSHIHIRO OKA et al.** *Current Opinion in Immunology,* 2008, vol. 20, 211-220
- **KAWAI et al.** *Nucleic Acids Research,* vol. 3, 103-4
- Japanese Pharmacopoeia

SEQUENCE LISTING FREE TEXT

SEQUENCE LISTING

<110>  NITTO DENKO CORPORATION

<120>  Vaccine composition for inhibiting apoptosis of CTL or blocking inhibition of CTL induction

<130>  F6527WO

<150>  JP2014-159002
<151>  2014-08-04

<160>  17

<170>  PatentIn version 3.5

<210>  1
<211>  9
<212>  PRT
<213>  Homo sapiens

<400>  1

Ala Tyr Ala Cys Asn Thr Ser Thr Leu
1               5

<210>  2
<211>  9
<212>  PRT
<213>  Homo sapiens

<400>  2

Glu Tyr Ile Leu Ser Leu Glu Glu Leu
1               5

<210>  3
<211>  9
<212>  PRT
<213>  Homo sapiens

<400>  3

Thr Tyr Leu Pro Thr Asn Ala Ser Leu
1               5

<210>  4
<211>  9
<212>  PRT
<213>  Homo sapiens

<400>  4

Ile Met Pro Lys Ala Gly Leu Leu Ile
1               5

<210>  5
<211>  9
<212>  PRT

<213> Homo sapiens

<400> 5

Val Leu Gln Glu Leu Asn Val Thr Val
1 5

<210> 6
<211> 9
<212> PRT
<213> Homo sapiens

<400> 6

Lys Val Phe Gly Ser Leu Ala Phe Val
1 5

<210> 7
<211> 9
<212> PRT
<213> Homo sapiens

<400> 7

Lys Val Ala Glu Ile Val His Phe Leu
1 5

<210> 8
<211> 9
<212> PRT
<213> Homo sapiens

<400> 8

Lys Ile Phe Gly Ser Leu Ala Phe Leu
1 5

<210> 9
<211> 9
<212> PRT
<213> Homo sapiens

<400> 9

Ser Thr Ala Pro Pro Val His Asn Val
1 5

<210> 10
<211> 9
<212> PRT
<213> hepatitis C virus

<400> 10

Asp Leu Met Gly Tyr Ile Pro Ala Val
1 5

<210> 11
<211> 9
<212> PRT
<213> hepatitis B virus

<400> 11

Trp Leu Ser Leu Leu Val Pro Phe Val
1 5

<210> 12
<211> 20
<212> DNA
<213> Unknown

<220>
<223> bacterial DNA

<400> 12
tccatgacgt tcctgacgtt 20

<210> 13
<211> 15
<212> PRT
<213> Mycobacterium tuberculosis

<400> 13

Phe Gln Asp Ala Tyr Asn Ala Ala Gly Gly His Asn Ala Val Phe
1 5 10 15

<210> 14
<211> 15
<212> PRT
<213> Artificial Sequence

<220>
<223> Peptide-25B

<400> 14

Phe Gln Asp Ala Tyr Asn Ala Val His Ala Ala His Ala Val Phe
1 5 10 15

<210> 15
<211> 13
<212> PRT
<213> Artificial Sequence

<220>
<223> PADRE

<220>
<221> MOD_RES
<222> (1)..(1)
<223> D-alanine

<220>

<221> MOD_RES
<222> (3)..(3)
<223> cyclohexylalanine

<220>
<221> MOD_RES
<222> (13)..(13)
<223> D-alanine

<400> 15

Ala Lys Ala Val Ala Ala Trp Thr Leu Lys Ala Ala Ala
1 5 10

<210> 16
<211> 8
<212> PRT
<213> Gallus gallus

<400> 16

Ser Ile Ile Asn Phe Glu Lys Leu
1 5

<210> 17
<211> 17
<212> PRT
<213> Artificial Sequence

<220>
<223> Synthetic peptide

<400> 17

Ile Ser Gln Ala Val His Ala Ala His Ala Glu Ile Asn Glu Ala Gly
1 5 10 15

Arg